(19) Europäisches Patentamt / European Patent Office / Office européen des brevets

(11) **EP 2 533 788 B1**

## (12) EUROPEAN PATENT SPECIFICATION

(45) Date of publication and mention
of the grant of the patent:
**16.12.2015 Bulletin 2015/51**

(21) Application number: **11740120.8**

(22) Date of filing: **08.02.2011**

(51) Int Cl.:
*A61K 9/00* (2006.01)     *A61K 47/02* (2006.01)
*A61K 35/16* (2015.01)    *A61K 35/18* (2015.01)
*A61K 35/57* (2015.01)    *C07K 14/745* (2006.01)
*A61K 38/36* (2006.01)    *A61K 38/48* (2006.01)
*G01N 33/49* (2006.01)    *G01N 33/86* (2006.01)

(86) International application number:
**PCT/SE2011/050144**

(87) International publication number:
**WO 2011/096887 (11.08.2011 Gazette 2011/32)**

---

(54) **STABLE SOLUTION**

STABILE LÖSUNG

SOLUTION STABLE

---

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.02.2010 US 302196 P
08.02.2010 SE 1050124**

(43) Date of publication of application:
**19.12.2012 Bulletin 2012/51**

(73) Proprietor: **Nordic Haemostasis AB
590 73 Ljungsbro (SE)**

(72) Inventors:
• **LINDAHL, Tomas
  S-587 31 Linköping (SE)**
• **FAXÄLV, Lars
  S-590 73 Ljungsbro (SE)**

(74) Representative: **Bokinge, Ole
Awapatent AB
Junkersgatan 1
582 35 Linköping (SE)**

(56) References cited:
EP-A1- 0 130 708     EP-A1- 0 434 377
WO-A1-89/11865      WO-A1-91/16913
WO-A1-97/04081      WO-A1-2004/007707
US-A- 4 203 891      US-A- 6 124 110

• OctaPharma Ltd: "Summary of product
  characteristics. Octaplex", OctaPharma Ltd ,
  October 2010 (2010-10), XP002699045, Retrieved
  from the Internet: URL:http:
  //www.medicines.org.uk/emc/medici ne/21897
  [retrieved on 2013-06-18]
• HAEMATOLOGIC TECHNOLOGIES INC.
  HAEMATOLOGIC TECHNOLOGIES INC.
  PRODUCTS: FACTOR XII DEFICIENT PLASMA
  (HAEMATOLOGIC TECHNOLOGIES, INC.
  CATALOG #FXII-ID) ESSEX JUNCTION, USA,
  XP008163901
• AMERICAN DIAGNOSTICA INC. PRODUCTS:
  HUMAN FACTOR XII DEFICIENT PLASMA
  (PRODUCT NO 212, LOT NO 061115, EXP.
  2009-11-16) 16 November 2009, STAMFORD,
  USA,
• HEMOSIL PRODUCTS: FACTOR XII DEFICIENT
  PLASMA XP008163903
• HELENA LABORATORIES PRODUCTS: FACTOR
  XII DEFICIENT SUBSTRATE PLASMA
  XP008163902

---

EP 2 533 788 B1

**Description**

TECHNICAL FIELD

The present invention relates to the field of blood coagulation. More precisely, it relates to a stable solution and its use as control material for coagulation analysis, and methods relating to coagulation analysis.

BACKGROUND OF THE INVENTION

Coagulation is a complex process by which blood forms clots. It is an important part of hemostasis (the cessation of blood loss from a damaged vessel), wherein a damaged blood vessel wall is covered by a platelet and fibrin-containing clot to stop bleeding and begin repair of the damaged vessel. Disorders of coagulation can lead to an increased risk of bleeding (hemorrhage) or clotting (thrombosis).

Coagulation is highly conserved throughout biology; in all mammals, coagulation involves both a cellular (platelet) and a protein (coagulation factor) component. The system in humans has been the most extensively researched and, therefore, the best-understood.

Coagulation begins almost instantly after an injury to the blood vessel has damaged the endothelium (lining of the vessel), this exposes or releases the membrane protein tissue factor that initiates a chain reaction. Platelets adhere to subendothelial subsytances such as collagen and von Willebrand factor, become activated and subsequently form a plug at the site of injury; this is called *primary hemostasis. Secondary hemostasis* occurs simultaneously: Proteins in the blood plasma, called *coagulation factors* or *clotting factors,* respond in a complex cascade to form fibrin strands, which strengthen the platelet plug.

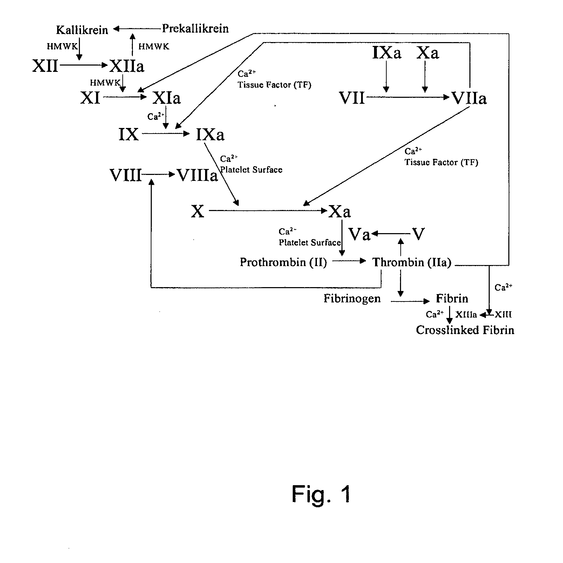

Figure 1 shows an overview of the coagulation system in humans adopted from Ramström, S, Thesis, 2001, Linköping University Medical Dissertation no. 776 (ISBN 91-7373-535-3.

The coagulation cascade of secondary hemostasis has two pathways, the *contact activation pathway* (formerly known as the intrinsic pathway), and the *tissue factor pathway* (formerly known as the extrinsic pathway), which lead to *fibrin* formation. The primary pathway for the initiation of blood coagulation is the *tissue factor* pathway. Both the *tissue factor* and *contact activation* pathways activate the final *common pathway* of factor X, thrombin and fibrin (see Figure 1).

*Testing the coagulation system*

Numerous tests are used to assess the function of the coagulation system, e.g. aPTT (activated partial thromboplastin time), PT (prothrombin time, used also to determine INR), fibrinogen testing (may be done by the Clauss method), platelet count, platelet function testing (often by PFA-1 00), TCT, bleeding time, mixing test (whether an abnormality of a patient is corrected if the patient's plasma is mixed with normal plasma), coagulation factor assays, antiphospholipid antibodies, D-dimer, genetic tests (e.g. factor V Leiden, prothrombin mutation G2021 0A), dilute Russell's viper nenom time (dRVVT), miscellaneous platelet funtion tests, thromboelastography (TEG or Sonoclot), euglobulin lysis time (ELT).

The contact activation pathway is initiated by activation of *contact factors* of plasma, and can be measured by a aPTT test.

The tissue factor pathway is initiated by exposure to the blood of or tissue factor or release of *tissue factor* (a specific cellular membrane protein), and can be measured by a PT test. PT results are often reported as a normalised ratio, i.e. International Normalised Ratio (INR) value.

*Disease and testings*

A disorder in the coagulation system may dispose to hemorrhage, thrombosis, and occasionally both, depending on the nature of the pathology. Thus, the need of an accurate testing and measuring is highly appreciated and the need of proper control material in the assessment of the coagulation system is apparent. Also, a proper handling of the control material by the care taking staff is of importance. In most cases, the point of care for coagulation tests are performed by either the patient themselves or by hospital staff where neither of the two groups being properly trained in laboratory skills. Today's control material is mostly based on freeze dried plasma comprising citrate buffer as buffering and as chelating agent. The freeze-dried plasma needs to diluted just before use and calcium needs to be added to restore the coagulation capacity of the freeze-dried plasma. Thus, the reconstituted solution is short lived and should be used within 4h to remain high quality. Further, improper handling of the control sample by the user (staff or patient) by accidentally extending time before use or by incorrect dilutions is a recognized problem which will affect the result of the assay and the following medication based on the result.

There is thus an urgent need to provide more stable and more reliable controls to coagulation assays, especially tests where the material analyzed is fresh, native human blood (i.e. with no additives made before analysis) to remove potential variations and unreliable results in assessing the coagulation system status in a patient due to possible dilution errors or expired control samples. Thus, the present invention addresses this need and interest.

SUMMARY OF THE INVENTION

The invention is defined by the appended independent claims. Embodiments are set forth in the dependent claims, in the attached drawings and in the following description.

An aspect of the present invention provides a stable solution comprising isolated factor II, VII and X and isolated plasma, wherein the factors are isolated in the isolated plasma and wherein the isolated plasma is depleted of coagulation factor XII, wherein the solution comprises $\geq 0.5$ mmol/L and $\leq 20$ mmol/L calcium and wherein the solution is stable, i.e. INR change of less than 15 %, for >4h at 2-8 °C and/or at RT.

An embodiment of the invention is wherein the depletion of coagulation factor XII is 95-100%.

A non-claimed embodiment is wherein the depletion of coagulation factor XII is 100% or close to 100%.

FURTHER EMBODIMENTS ARE WHEREIN THE STABLE SOLUTION IS WITH THE PROVISO THAT SAID SOLUTION DOES NOT COMPRISE ANY CALCIUM CHELATING AGENT.

Further non-claimed embodiments are wherein the solution comprises physiological or above physiological amounts of calcium.

Further embodiments are wherein the plasma is depleted of platelets.

In a non-claimed embodiment the stable solution is being stable for at least 1 week, such as 1 week, 2 weeks, 3 weeks, 4 weeks, 5 weeks, 6 weeks, 7 weeks, 8 weeks, 2 months, 3 months, 4 months, 5 months, 6 months, 7 months, 8 months, 9 months 10 months, 11 months, 12 months or even longer at 2-8 °C or at RT.

A further aspect of the present invention is the use of the stable solution of the first aspect as control material for coagulation analysis in assays/tests selected form a group consisting of prothrombin time (PT), mixing test, coagulation factor assays, thromboelastography euglobulin lysis time (ELT) or activated thromboplastin time (aPTT) with the proviso that no additional calcium is added to the solution upon use.

A non-claimed embodiment is a method of producing a stable solution, the method comprising

a) isolating plasma

b) depleting the plasma of factor XII

c) optionally, lyophilizing the isolated and factor XII-depleted solution.

One alternative non-claimed embodiment is a method assessing coagulation system status in a subject, the method comprising

a) providing a stable solution according to the present invention,

b) providing a blood sample from a subject,

c) measuring status of the coagulation system of said subject,

d) analysing status of the coagulation system of said patient,

e) compare status of the coagulation of said subject to a positive and/or negative standard, thereby assessing the coagulation system status in said patient.

The positive and/or negative standard form reference points, and, if suitable, may be plotted in a standard curve. The standard curve and each respective reference points may be made by measuring separate reference points made of a mix of plasma without factor XII (i.e. depleted plasma or plasma from a factor XII deficient subject) - however with all other complement factors present - and a pooled normal plasma comprising factor XII. The deficient plasma and the normal plasma is mixed in different proportions to form different reference points. When measured, they may be plotted in a diagram to form a standard curve.

In a non-claimed embodiment the subject is a human subject.

In yet a non-claimed embodiment the method further comprises wherein the blood sample from a subject is mixed with the stable solution according to the invention before measuring and analyzing the status of the coagulation system.

Further aspects of the present invention encompass a kit comprising the stable solution according to the first aspect.

BRIEF DESCRIPTION OF FIGURES

**Fig. 1** shows an overview of the coagulation system in humans (adopted from Ramström, S, Thesis, 2001, Linköping University Medical Dissertation no. 776 (ISBN 91-7373-535-3). The coagulation cascade of secondary hemostasis has two pathways, the *contact activation pathway* (formerly known as the intrinsic pathway), and the *tissue factor pathway* (formerly known as the extrinsic pathway), which lead to *fibrin* formation. The primary pathway for the initiation of blood coagulation is the *tissue factor pathway.* Both the *tissue factor* and *contact activation* pathways activate the final *common pathway* of factor X, thrombin and fibrin.

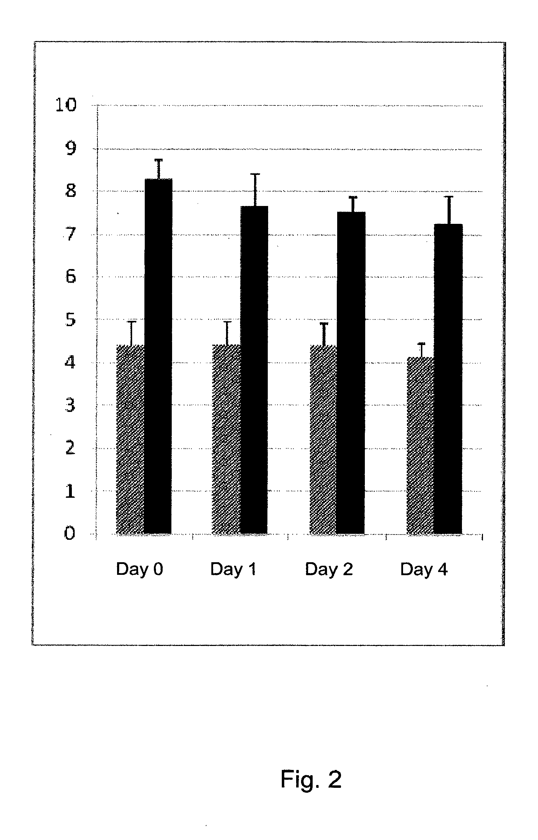

**Fig. 2** shows factor XII-activity in kaolin treated (striped) and titanium dioxide (solid black) treated plasma at day 0, 1, 2, and day 4. Results are calculated as substrate cleavage rate.

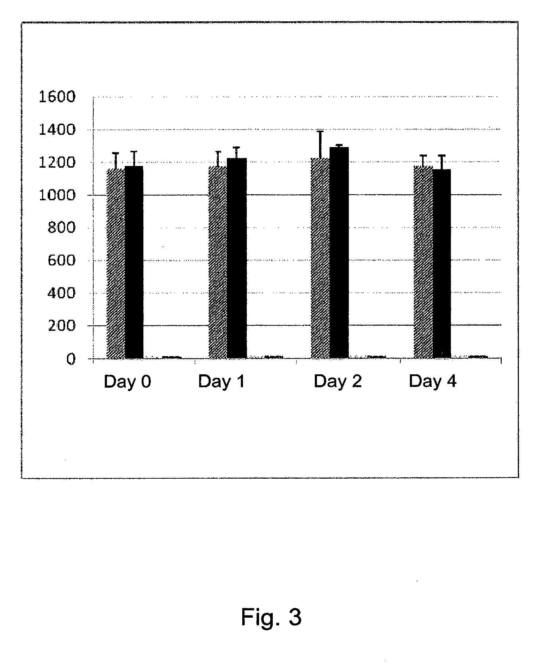

**Fig. 3** shows thrombin generation calculated as endogenous thrombin potential (ETP) presented as mean + SD after 0, 1, 2 and 4 days. Data represents incubation with titanium dioxide during 5 minutes (striped) and 60 minutes (solid black), and incubation with kaolin during 5 minutes (white with black dots) and 60 minutes (black with white dots) minutes respectively.

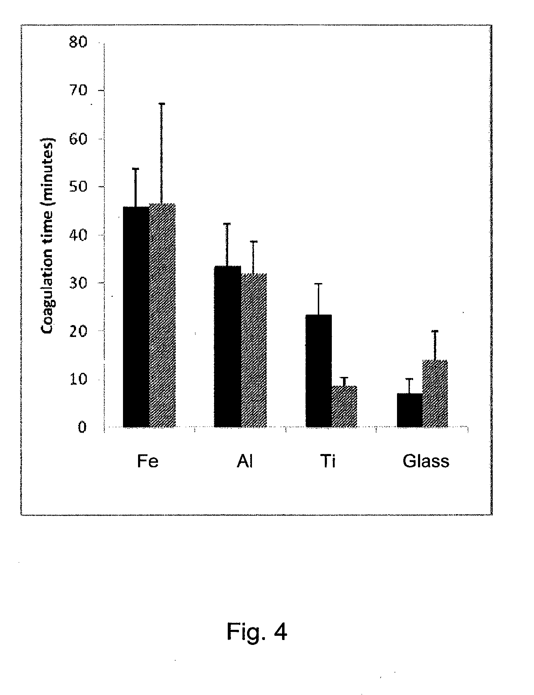

**Fig. 4** shows coagulation time at the material surface using plasma from four different donors. Data is presented as mean + SD for coagulation times when measured at Fe, Al, Ti, and glass surfaces. The materials were either measured directly (solid black) or after incubation in citrated plasma during 60 minutes followed by washing in HEPES (striped).

DETAILED DESCRIPTION

*Definitions*

As used herein, "stable" in relation to a solution or composition is intended to mean that the solution or composition is not readily decomposing or changing from one state of matter to another and not undergoing spontaneous change.

As used herein, "lyophilization", "freeze-drying" or "cryopreservation" is intended to mean a dehydration process typically used to preserve a perishable material or make the material more convenient for transport. Lyophilization works by freezing the material and then reducing the surrounding pressure and adding enough heat to allow the frozen water in the material to sublime directly from the solid phase to gas.

"Subject" as used herein, means any mammal including human having or suspected of having a disease. "Subject" as used herein denotes a mammal, such as a rodent, a feline, a canine, and a primate. Preferably a subject according to the invention is a human.

"At least one" as used herein means one or more, i.e. 1, 2, 3, 4, 5, 6, 7, 8, 9, 10 etc.

"Detection", "detect", "detecting" as used herein includes qualitative and/or quantitative detection (measuring levels) with or without reference to a control, and further refers to the identification of the presence, absence, or quantity of a given blood coagulation process.

As used herein, the singular forms "a", "and", and "the" include plural referents unless the context clearly dictates otherwise. Thus, for example, reference to "an antibody" includes a plurality of such antibodies.

As used herein "depletion" is intended to mean removal of said component or factor, partially or fully, or in any other way inactivating its activity partially or fully e.g. by adding inhibitors to said factors, which inactivates directly or indirectly the activity of said factor or component. Similarly, a "depleted solution" is absent fully or partially of a certain factor or component. As used herein a "depleted solution" also embraces a functional depletion or inactivation, fully or partially, of a factor or a component. This functionally depletion or inactivation may be directly or indirectly by the addition of e.g. inhibitors.

"Sensitivity", as used herein in the context of its application to blood coagulation method or assays, refers to the proportion of all subjects that are correctly identified as such.

"Treatment" as used herein is defined as the management of a patient through medical or surgical means. The treatment improves or alleviates at least one symptom of a medical condition or disease and is required to provide a cure. The term "treatment outcome" or "outcome of treatment" as used herein is the physical effect upon the patient of the treatment.

The term "algorithm" as used herein refers to a mathematical formula that provides a relationship between two or more quantities. Such a formula may be linear, or nonlinear, and may exist as various numerical weighting factors in computer memory.

"Monoclonal antibody" or "mAb" as used herein refers to an antibody of a single amino acid composition, that is directed against a specific antigen and that is produced by a single clone of B cells or hybridoma.

"Polyclonal antibody" as used herein refers to an antibody that is directed against a specific antigen that is derived from different B-cell lines.

"Fab" as used herein refers to an antibody fragment having a molecular weight of about 50,000 Da and antigen binding activity, in which about a half of the N-terminal side of H chain and the entire L chain, among fragments obtained by treating IgG with a protease, papaine, are bound together through a disulfide bond.

"F(ab')$_2$" as used herein refers to an antibody fragment having a molecular weight of about 100,000 Da and antigen binding activity, which is slightly larger than the Fab bound via a disulfide bond of the hinge region, among fragments obtained by treating IgG with a protease, pepsin.

"Fab'" as used herein refers to an antibody fragment having a molecular weight of about 50,000 Da and antigen binding activity, which is obtained by cutting a disulfide bond of the hinge region of the F(ab')2.

As used herein, a single chain Fv ("scFv") polypeptide is a covalently linked VH::VL heterodimer which is usually expressed from a gene fusion including VH and VL encoding genes linked by a peptide-encoding linker. The human scFv fragment of the invention includes CDRs that are held in appropriate conformation, preferably by using gene recombination techniques.

"Hybridoma" as used herein denotes a cell, which is obtained by subjecting a B cell prepared by immunizing a non-human mammal with an antigen to cell fusion with a myeloma cell derived from a mouse or the like which produces a desired monoclonal antibody having an antigen specificity.

As revealed above, the present invention provides a stable control sample solution. The solution according to the invention is more stable compared to in the art available solutions. Further, the stable control sample solution according to the invention is liquid at 2-8°C, has a good stability at 2-8°C and even at higher temperature such as room temperature (i.e ca 23°C), and is ready to use. Thus, no additional dilution steps, e.g. dilution in an exact volume of liquid are required nor necessary, thus reducing and/or eliminating errors due to dilutions and human hand.

Further, no additional calcium ions need to be added to the stable control sample solution according to the invention upon use. Even further, said stable control solution according to the invention is also stable in room temperature. E.g. the stability in room temperature is >4h, such as 5h, 10h, 20h, 1 day, 2 days, 3 days, 4 days, 5 days, 6 days, 7 days, i.e. a week, 2 weeks or even longer, such as 1 month, 2 months, 3 months, 4 months 5 months or even 6 months or a year room temperature. At 2-8°C, such as e.g. at 2 or 4°C the stability is even longer, such as 6 months, 7, 8, 9, 10, 12, 13, 14, 15, 16, 17, 18, 19, 20, 21, 22, 23 or even 24 months.

Thus, in one aspect the present invention provides stable solution comprising isolated plasma and wherein the isolated plasma is depleted of coagulation factor XII. Said stable solution comprises physiological or above physiological amounts of calcium. Said stable solution is further stable for at least 4h at 2-8°C or RT, such as 5h, 10h, 10h, 20h, 1 day, 2 days, 3 days, 4 days, 5 days, 6 days, 7 days, i.e. a week or even 2 weeks or even longer, even longer, such as 1 month, 2 months, 3 months, 4 months 5 months or even 6 months or a year room temperature. At 2-8°C, such as e.g. at 2 or 4°C the stability is even longer, such as 6 months, 7, 8, 9, 10, 12, 13, 14, 15, 16, 17, 18, 19, 20, 21, 22, 23 or even 24 months. Thus, the stable solution comprising isolated plasma and wherein the isolated plasma is depleted of coagulation factor XII, is with the proviso that no additional calcium is being added upon use and, further, that it is stable at >4h at 2-8°C or at RT.

Depletion of coagulation factor XII may be done according to in the art known techniques. One method is to use immunoadsorption of factor XII. Another method is immunodepletion using antibodies to factor XII as described in Braat et al (Eur. J. Biochemistry, 1999, 263:904-911) of factor XII from the plasma using immunoaffinity chromatography (see e.g. Current Protocols in Immunology, ISBN 1934-3671, Chapter 8, unit 8.2, 2007, by John Wiley and Sons, Inc). Antibodies capable of binding specifically to factor XII may be done according to known techniques in the art. Examples of antibodies to factor XII are given in WO89/11865.

Assays to measure the amount of remaining factor XII after purification are given below and in e.g. Jones et el. (Blood Coagul Fibrinolysis, 1998, 9(2):183-7), Stürzebecher, J. et al., (Thromb Res., 1989, 55(6):709-15)., and in Tankerslay et al. (Blood, 1983, 62:488-456).

Example of suitable assays for quantification of factor XII are different immunoassays, such as e.g. an ELISA (Enzyme Linked ImmunoSorbent Assay) assay. An ELISA utilises at least one antibody binding specifically to Factor XI Antibodies to human Factor VII are available from commercial sources such as e.g. Abcam, AbD Serotech, Thermo Scientific Pierce Antibodies, Acris Antibodies GmbH, AgriSera, AMERICAN DIAGNOSTICA, etc. Immuno assays, such as ELISA assays to identify and even quantify a protein, such as factor XII, are well known in the art and described in e.g. Antibodies: A laboratory Manual, Chapter 14, (Cold Spring Harbor Laboratory Press, New York, 1988, by Harlow, E and Lane, D.

In brief, factor XII activity in depleted plasma may further be measured by adding plasma known to be depleted in factor XII (FXII⁻ control plasma) but that comprises all other relevant factors for coagulation. When calcium is added the coagulation is starting. The clotting time may be measured by change in turbidity for example at 671 nm.

By "reacting specifically with" as used herein it is intended to equal "capable of binding selectively" or "binding specifically to". As used herein the expressions are intended to mean that the antibody or antigen-binding fragment, or variant, fusion or derivative thereof, including any antibody derived binding moiety, which is capable of binding to an antigen of a molecule and further which binds at least 10-fold more strongly to factor XII than to another protein for example at least 50-fold more strongly or at least 100- fold more strongly. The binding moiety may be capable of binding selectively to the protein under physiological conditions, e.g. in vivo. Suitable methods for measuring relative binding strengths include, immunoassays, for example where the binding moiety is an antibody (see Harlow & Lamp; Lane, "Antibodies: A Laboratory", Cold Spring Habor Laboratory Press, New York, which is incorporated herein by reference). Alternatively, binding may be assessed using competitive assays or using Biacore® analysis (Biacore International AB, Sweden).

Antibodies binding specifically to factor XII to be used in an immunodepletion method or immunoaffinity chromatography may be whole antibodies or fragments thereof, e.g. antigen-binding fragment, or variant, fusion or derivative thereof as long as they are capable of binding to the desired protein *in vitro.* Such binding specificity may be determined by methods well known in the art, such as e.g. ELISA, immunohistochemistry, immunoprecipitation, Western blots, chromatography and flow cytometry using transfected cells expressing the all subunit or a heterodimer thereof (see Examples). Examples of how to measure specificity of an antibody is given in e.g. Harlow & Lane, "Antibodies: A Laboratory", Cold Spring Habor Laboratory Press, New York, which is incorporated herein by reference.

By "antibody" we include substantially intact antibody molecules of any species such as rodents, e.g. murine, rat, guneapig, or non-rodents such as rabbit, goat, sheep, dog, pig, camel, dromedary, donkey, horse or chicken, as well as chimaeric antibodies, humanized antibodies, human antibodies (wherein at least one amino acid is mutated relative to the naturally occurring human antibodies), single chain antibodies, bispecific antibodies, antibody heavy chains, antibody light chains, homodimers and hetero-dimers of antibody heavy and/or light chains, and antigen binding fragments and derivatives of the same. For example, the antibody may be a monoclonal antibody.

Antigenic specificity is conferred by variable domains and is independent of the constant domains, as known from experiments involving the bacterial expression of antibody fragments, all containing one or more variable domains. These molecules include Fab-like molecules (Better et al (1988) Science 240, 1041); Fv molecules (Skerra et al (1988) Science 240, 1038); single-chain Fv (ScFv) molecules where the $V_H$ and $V_L$ partner domains are linked via a flexible oligopeptide (Bird et al (1988) Science 242, 423; Huston et al (1988) Proc. Natl. Acad. Sd. USA 85, 5879) and single domain antibodies (dAbs) comprising isolated V domains (Ward et al (1989) Nature 341, 544). A general review of the techniques involved in the synthesis of antibody fragments which retain their specific binding sites is to be found in Winter & Milstein (1991) Nature 349, 293- 299.

Thus, by "antigen-binding fragment" we mean a functional fragment of an antibody that is capable of binding specifically to factor XII(e.g. Fab fragments, Fab' fragments and F(ab)2 fragments), single antibody chains (e.g. heavy or light chains), single variable domains (e.g. VH and VL domains) and domain antibodies (dAbs, including single and dual formats; i.e. dAb-linker-dAb).

The term 'antibody' also includes all classes of antibodies, including IgG, IgA, IgM, IgD and IgE. In one embodiment, however, the antibody is an IgG molecule, such as an IgGI, IgGI, IgG3, or IgG4 molecule.

Methods of generating antibodies and antibody fragments are well known in the art. For example, antibodies may be generated via any one of several methods which employ induction of in vivo production of antibody molecules, screening of immunoglobulin libraries (Orlandi. et al, 1989. Proc. Natl. Acad. Sci. U.S.A., vol 86, pages 3833-3837; Winter et al, 1991, Nature 349:293-299, which are incorporated herein by reference) or generation of monoclonal antibody molecules by cell lines in culture. These include, but are not limited to, the hybridoma technology, the human B-cell hybridoma technology, and the Epstein-Barr virus (EBV)-hybridoma technology (see Kohler et al, 1975. Nature 256:4950497; Kozbor et al, 1985. J Immunol. Methods 81 :31-42; Cote et al, 1983. Proc. Natl. Acad. Sci., USA 80:2026-2030; Cole et al, 1984. Mol Cell. Biol. 62:109-1 20, which are incorporated herein by reference).

Antibodies may be done by immunization where the whole protein or a suitable fragment thereof can be injected into non-human mammals (such as mice or rabbits), followed by boost injections, to produce an antibody response. Serum isolated from immunized animals may be isolated for the polyclonal antibodies contained therein, or spleens from immunized animals may be used for the production of hybridomas and monoclonal antibodies.

In one example, a monoclonal antibody to one of the proteins can be prepared from murine hybridomas according to the classical method of Kohler and Milstein {Nature, 256:495, 1975) or derivative methods thereof. Briefly, a mouse (such as Balb/c) is repetitively inoculated with a few micrograms of the selected protein or peptide fragment thereof or a suitable carrier conjugate thereof over a period of a few weeks. The mouse is then sacrificed, and the antibody-producing cells of the spleen isolated. The spleen cells are fused by means of polyethylene glycol with mouse myeloma cells, and the excess un-fused cells destroyed by growth of the system on selective media comprising aminopterin (HAT media). The successfully fused cells are diluted and aliquots of the dilution placed in wells of a microtiter plate where growth of the culture is continued.

Antibody-producing clones are identified by detection of antibody in the supernatant fluid of the wells by immunoassay procedures, such as ELISA, as originally described by Engvall (Enzymol., 70:419, 1980), and derivative methods thereof.

Selected positive clones can be expanded and their monoclonal antibody product harvested for use.

Antibodies may be purified before use. Purification of antibodies are done using techniques available in the art and described in e.g. "Monoclonal Antibodies: Amanual of techniques", H Zola (CRC Press, 1988) and in "Monoclonal Hybridoma Antibodies: Techniques and Applications", J G R Hurrell (CRC Press, 1982), which are incorporated herein by reference.

The antibody or antigen-binding fragment or derivative thereof may also be produced by recombinant means. Suitable monoclonal antibodies to selected antigens and proteins may be prepared by known techniques, for example those disclosed in "Monoclonal Antibodies: A manual of techniques", H Zola (CRC Press, 1988) and in "Monoclonal Hybridoma Antibodies: Techniques and Applications", J G R Hurrell (CRC Press, 1982), and "Antibodies: A Laboratory Manual", Cold Spring Harbor Laboratory, New York, which are incorporated herein by reference.

Antibody fragments can also be obtained using methods well known in the art (see, for example, Harlow & Lane, 1988, "Antibodies: A Laboratory Manual", Cold Spring Harbor Laboratory, New York, which is incorporated herein by reference). For example, antibody fragments may be prepared by proteolytic hydrolysis of the antibody or by expression in E. coli or mammalian cells (e.g. Chinese hamster ovary cell culture or other protein expression systems) of DNA encoding the fragment.

Alternatively, antibody fragments can be obtained by pepsin or papain digestion of whole antibodies by conventional methods.

Thus, the depletion of coagulation factor XII may be 95-100%, e.g. 95, 96, 97, 98, 99, 99.9% compared to original levels of the blood when withdrawn from a patient.

Accordingly, further embodiments of solutions according to the invention are wherein the depletion of coagulation factor XII is 95-100%, e.g. 95, 96, 97, 98, 99, 99.9%.

In addition to depletion of coagulation factor XII, at least one further coagulation factor of the contact activation system may be depleted. Examples of further coagulation factors are factor XI, prekallikrein, high-molecular-weight kininogen (HMWK, also known as the Williams-Fitzgerald-Flaujeac factor or the Fitzgerald factor or the HMWK-kallikrein factor).

Accordingly, further embodiments of said solutions of the invention are wherein at least one further coagulation factor of the contact activation system is depleted in addition to factor XII. In one further embodiment of said solutions according to the invention, factor XI is depleted in addition to factor XII. The depletions of each of factor XII and factor XI may be at the same level, e.g. about 95-100%, however, numerical not identical. This means that for example, factor XII may be depleted to about 99% and factor XI may be depleted for about 96%, or the opposite, i.e. within the range but with different numerical values within said range. The numerical value may, of course, also be the same. If two or more factors are depleted, it is appreciated that not all factors needs to be depleted fully, but that trace amounts of each factor may remain. E.g. if Factor XII and Factor XI are both depleted, they may both be depleted to about 95-96% and still deliver a very stable solution according to the invention.

Thus, in still further embodiments of said solutions, factor XII and factor XI are each depleted about 95-100%, e.g. each about 95, 96, 97, 98, 99, 99.9% respectively.

Still further embodiments of said stable solutions of the invention are where in the depletion of coagulation factor XII is 100%, as measured by e.g. ELISA originally described by Engvall (Enzymol., 70:41 9, 1980), and derivative methods thereof.

The amount of factor XII may also be measured as amount of activity or residual activity after depletion, apart from the quantitative measurements mentioned herein. Activity of coagulation factor XII may be measured by any coagulation factor assay reflecting the activity of Factor XII, such as the ones described herein or any other assay known to someone skilled in the art of coagulation assays. The most common assay is the APTT-based methods with FXII-deficient plasma, for example the IL method for ACL Top (Milan, Italy). There are also methods using chromogenic substrates (see e.g.Practical application of a chromogenic FXIIa assay. Zhuo R, Vogler EA.Biomaterials. 2006 Oct;27(28):4840-5.An automated chromogenic peptide substrate assay for coagulation factor XII. Jones DW, Gallimore MJ, Winter M. Blood Coagul Fibrinolysis. 1998 Mar;9(2):183-7)

Accordingly, further embodiments of said solutions of the invention are wherein the activity of coagulation factor XII is zero (0) or close to zero.

The zero-activity of Factor XII may be achieved, apart from depletion, by addition of a Factor XII inhibitor. Such a Factor XII-inhibitor may be a direct or an indirect inhibitor. A direct inhibitor acts directly on Factor XII. An indirect Factor XII inhibitor inhibits in the Factor XII-cascade as shown in figure 1. Examples of suitable direct or indirect factor XII inhibitors are corn trypsin inhibitor, a direct inhibitor of factor XII.

Thus, as described above, the stable solution according to the invention is thus a stable solution wherein the internal, normally working, coagulation cascade is non-functioning or mal-functioning due to depletion or inactivation of factor XII. The way to render the normally working coagulation cascade non-working may be by e.g. depletion of coagulation factor XII or by a direct or indirect inhibition as described herein.

Still a further way to render the normally working coagulation cascade non-working is by addition of inhibitors acting directly or indirectly to Factor XII, e.g. such as mentioned herein or otherwise known in the art, or by addition of antibodies that may act, directly or indirectly, to block the activity of Factor XII.

The normal coagulation cascade may also be non-working by hereditary deficiency. However, unlimited access or large quantities to isolated blood plasma from such donors is not convenient since the condition is rare, total FXII-deficiency in less 1/10,000 and FXI-deficiency is even rarer.

Thus, further embodiments of the solutions of the invention are wherein the stable solutions according to the invention are depleted of factor XII, or in any other way with a non-working, or mal-functioning or dis-functioning coagulation cascade as described herein. The non-working, or mal-functioning or dis-functioning coagulation cascade is identified by comparison to a coagulation cascade in a normal, healthy subject. A suitable assay for assessing a normal coagulation cascade is as exemplified herein or in any other way assessed by a coagulation assay known in the art (see e.g. Klinisk Biokemi i Norden". Special issue 2008: Coagulation. Eds Strandberg K, Hillarp A. Laboratory techniques in thrombosis - a manual. 2nd revised edition of ACAT Assay procedures. Eds Jespersen J, Bertina RM, Haverkate F. Kluwer Academic Publishers 2000.

Thus, as used herein, depleted of Factor XII or a factor XII-depletion, is to embrace, except from depletion of factor XII as such, also depletion of factor XII activity by direct or indirect means. Examples of direct or indirect means for depletion of Factor XII activity is by e.g. the addition of factor XII inhibitors acting direct or indirect or by the additions of antibodies binding specifically to Factor XII or to any other factor in the coagulation cascade acting in the same downstream cascade as Factor XII (see fig 1) as mentioned herein. Examples of other factors acting in the same downstream cascade as factor XII are factor XI or prekallekrein, high-molecular-weight kinonogen. Further factors are given in figure 1. For coagulation assays applicable for Coaguchek instruments and similar, factor FX and downstream factors cannot me removed

As described herein, said stable solutions of the invention with factor XII depleted or inactivated are stable solutions with calcium levels from about 0.5 mmol/L and above, such as physiological levels or above of calcium. Since most blood is withdrawn with a calcium chelator, the calcium levels needs to be instantly adjusted in conjunction with, preferably after, the depletion and/or inactivation of factor XII to avoid coagulation. Said solution will then remain stable >4h in RT and 2-8°C.

Normally, when blood is withdrawn, and especially when withdrawn for a subsequent coagulation analysis, a calcium chelator, i.e. a chelating agent or compound, is added, usually citrate, in an excess. E.g. for citrate if added to a final concentration of 10 mmol/L to the withdrawn blood or plasma with a physiological calcium concentration of 1,3 mmol/L the final concentration of free calcium will be very low, if any (see e.g. Rånby et al., Clinical Chemistry, 45:8, pg 1176-1180, especially figure 1)

Further embodiments are where in no further calcium is added to the stable solution according to the invention. This may be the case when the blood withdrawn is from a patient with an inherent deficiency of Factor XII activity or expression. Thus, for patients or blood donors being deficient for Factor XII or any of the other factors mentioned herein, no calcium chelator is needed to the depleted blood and its subsequent isolated plasma and the calcium levels will remain physiological and the solution stable.

Thus, accordingly, embodiments of the solutions according to the invention are wherein said stable solutions as described herein is with the proviso that said solution does not comprise any calcium chelating agent.

However, normally, control material for coagulation assays are anti-coagulated blood (i.e. with a chelator added upon withdrawal of the blood) based on blood cell plasma and wherein further platelets has been removed. Removal of platelets may be done by centrifugation followed by filtration through a filter with pore size 0.25 µm (MiniSart™, Sartorius, Germany) in order to remove all platelets and fragments thereof.

The anticoagulant normally added to control material in the art is calcium chelating substances such as e.g. citrate or isocitrate. This is also the case of the stable solution of the present invention. Usually the anti-coagulant, e.g. a chelator such as citrate, is added 1/10 v/v at a concentration of about 0.105 mol/L to 0.13 mol/L if citrate is used. If isocitrate is used a slightly higher concentration may be used, such as e.g. 0.3 mol/l. Thus, in the normal case when an anti-coagulant or chelator is added to the stable solution of the invention upon its preparation, the calcium levels needs to be adjusted to physiological or above physiological to be stable as described herein. Thus, to make the solution stable, said levels of calcium must be physiological of above upon its preparation of the stable solution. However, the further need for adding calcium or other factors upon use of the solution, as is the case of all known control material for coagulation assays, is thus not needed. The stable solution will accordingly have a much easier performance of the end user being a patient itself or day care personnel to a patient. Less handling, dilution and extra addition upon use will minimize errors known to exist due to this extra handling of existing coagulation control material and will give a much more correct coagulation values for the patient. More correct coagulation values will, accordingly, give more accurate care and medicament to the patient as such.

Factor XII is then normally removed by immunoadsorption (immunoaffinity chromatography) that may be repeated. Also, other components of the contact activation system may be removed by immunoadsorbtion, e.g. coagulation factor XI (described in e.g. WO2008/081025), factor X (described in e.g. WO95/01571) or prekallikrein (WO2004/047859). Also, the stability may be improved by removal of all phospholipid containing membranes (from blood cells).

As an alternative, one may use blood from a factor XII deficient donor. However, the supply thereof is very limited. Purification of factor XII deficient plasma may be done by collection of blood by venipuncture from donors with inherent factor XII deficiency.

According to one aspect of the invention, the stable solution described herein comprises ≥ 0.5 mmol/L free calcium, such as physiological or even above physiological amounts of calcium. Physiological amount of ionised calcium is about 1.17- 1.29 mM (NORIP). Thus, further embodiments are wherein the amount of ionised calcium is about 0.5, 0.75, 1, 1.1, 1.2, 1.3, 1.4, 1.5, 1.6, 1.7, 1.8, 1.9, 2, 5, 10, 15, or even 20 mM. Thus, the calcium levels in the stable solution of the invention may be about 0.1-0.5 mM or above. In any case, the stable solution of the invention does not have a calcium level of 0.5 mmol/L or below. Accordingly, further embodiments encompass wherein the calcium levels is at least not below physiological levels or above physiological levels.

In citrated plasma, prepared from blood with conventional addition of 1/10 volume of citrate 0.11 or 0.13 mol/L as anticoagulant the final concentration of free calcium is extremely low since citrate forms a 1:1 complex with ionised calcium. Since the physiological level is 1-2 mmol/L the citrate is added in large excess when blood is withdrawn from a subject. The concentration of inoised calcium has been reported to be about 1,25 mmol/L (see e.g. - Laurells Klinisk kemi. Ed Nilsson-Ehle P. Studentlitteratur 2003, and Brown EM, MacLeod J. Extracellular calcium sensing and extracellular calcium signalling. Physiol Rev 2001;81:239-97+ Larsson L, Magnusson P. Ionized calcium or corrected total calcium? J Bone Miner Res 2003;18:1554-5).

Further embodiments of the stable solutions according to the invention are wherein the plasma of the stable solution is depleted of platelets. Removal of platelets may be done as described above, i.e. by centrifugation followed by filtration through a filter with pore size 0.25 μm (MiniSart™, Sartorius, Germany) in order to remove all platelets and fragments thereof.

Further embodiments are wherein the stable solution further comprises isolated and washed erythrocytes. Erythrocytes may be isolated by standard venipuncture blood collection from healthy donors. It is of importance to add washed erythrocytes to remove all possible factor XII in the preparation. Further, as much as possible of leucocytes and platelets should be removed. This is usually done by first making a platelet-rich plasma by centrifugation and then the subsequent removal of the plasma (including the platelets) and the buffy coat. The buffy coat is the fraction of an anticoagulated blood sample after centrifugation at about 800xg for about 20 minutes that contains most of the white blood cells and platelets. After centrifugation one can distinguish a layer of clear fluid (the plasma), a layer of red fluid containing most of the red blood cells (erythrocytes), and a thin layer in between, making up less than 1% of the total volume of the blood sample, the buffy coat with most of the white blood cells and platelets.

The amount of washed erythrocytes added to the stable solution of the invention is primarily to make the stable solution usable as control material for coagulation analysis (assays) more similar to blood since blood is the sample to be tested. If washed erythrocytes are not added, the stable solution of the invention will differ substantially from the blood tested from the patients and thus a calibration and addition of a factor needs to be done by an algorithm. This is known in the art and done in different analysis such as e.g. the instrument of Roche (CoaguCheck XS System, CoaguCheck XS Plus and CoaguCheck XS Pro System). Other examples of re-calculation by a factor e.g. via a coding chip is also convenient and known for e.g. Coagucheck XS system (see e.g. www.rochediagnostics.se/content/se/coaguchcek/coaguChekXS.html), ProTime® Microcoagulation System www.protimesystem.com, and the Abbott i-STAT system (see e.g. www.abbottpointofcare.com).

Thus, one embodiment is wherein washed erythrocytes are added to the stable solution of the invention. The washed erythrocytes are added to the stable solutions of the invention are in an amount equaling the amount of erythrocytes normally in blood, i.e. about 45%, such as 40%, 41%, 42%, 43%, 44%, 45%, 46%, 47%, 48%, 49% or even about 50% of the total blood. In further embodiments, the amount if erythrocytes added to the stable solutions of the invention is about 1%, 2%, 5%, 10%, 15%, 20%, 25%, 30%, 35%, 40%, 45%, 50%, 55, or even about 60%.

The stable solution according to the invention may thus further comprise erythrocytes to resemble fresh blood samples and thus, no further re-calculation or calibration of assay results or data via e.g. an algorithm on a coding chip or mere re-calculation due to differences between plasma and blood is needed when performing the blood coagulation analysis (assay).

The stable solution according to the invention is being stable for >4h at 2-8 °C or even >4 at RT, which is, of course, more demanding. The stable solution of the invention is more stable than solutions know in the art which needs to be used normally within 4h of reconstitution. This is not needed for the stable solution of the invention. In further embodiments, the stable solution according to the invention is being stable for at least 1 week at RT or at 2-8 °C, such as 1 week, 2 weeks, 3 weeks, 4 weeks, 5 weeks, 6 weeks, 7 weeks, 8 weeks, 2 months, 3 months, 4 months, 5 months, 6 months, 7 months, 8 months, 9 months 10 months, 11 months, 12 months or even longer as described and exemplified further herein.

The stabile solution of the invention is also stable in room temperature for > 4h, such as 5, 6, 7, 8, 9, 10h, or even 13, 14, 15, 16, 17, 18, 19, 20 or enen 24h, i.e. more than a day, 2 days, 3, 4, 5, 6, 7, 8, 9, 10, 11, 12, 13, 14 days or 2 weeks, 3, 4, 5, 6, 7, 8, 9, 10 weeks or even 1 month, 2 months, 3 months, 4 month, 5 months, or longer all in room temperature (RT) as described and exemplified further herein.

The stable solution according to the invention is wherein the isolated plasma is any mammal plasma, such as bovine, pig, feline, canine, rabbit, murine or human plasma.

In one further aspect of the present invention, the stable solution comprises isolated human factors II, VII and X, i.e. three K-vitamin dependent factors. It may also comprise factor V. If using isolated human factor II, VII and X, another source of plasma than human may be used, such as e.g. avian plasma.

Thus, in further embodiments, the stable solution of the invention comprises avian plasma with isolated human factors II, VII and X, i.e. three K-vitamin dependent factors, and optionally also factor V to increase the reaction rate. If so, any source of plasma may be used as plasma base, e.g. rabbit or bovine plasma. However, if using another source than human plasma, such as e.g. bovine or rabbit, then it is very important to avoid contamination with factors from the contact activation system, i.e. prekallekrein, HMW kininogen, factor XI and factor XII. Thus, the isolated factors II, VII, and X needs not have any detectable amounts of at least one of the factors from the contact activation system. Said factors, e.g. human factors II, VII and X, i.e. the K-vitamin dependent factors needed for a control material for prothrombin time or control material for analysis of any of these factors, may also be recombinant and added to e.g. avian or plasma from mammals. The latter plasma must, however, be depleted of at least one of the contact activating factors.

For control materials intended for prothrombin time methods dependent on generation of fibrin from fibrinogen (to make a clot), fibrinogen must be added if pure factors are used to produce the control material. Examples of such methods are i-STAT (Abbott, US), HemoSense INRatio System (Hemosense, US) and Hemochron® Jr. Signature Whole Blood Microcoagulation System (manufactured by ITC, US.). Suitable levels of fibrinogen to be added to the stable solution is to generate about 0,5 g/L final concentration or above, such as 1, 1,5, 2, 2,5, 3, 3,5, 4, 4,5, g/L of fibrinogen in final concentration.

In further embodiments the stable solution according to the invention is wherein the isolated plasma is human plasma.

In further embodiments, the bovine plasma is from cow.

In further embodiments, the stable solution of the invention comprises avian plasma with isolated human factors II, VII and X, i.e. K-vitamin dependent factors. If so, any source of plasma may be used as plasma base, e.g. avian or bovine plasma, however, then it is very important to avoid contamination with factors from the contact activation system, i.e. prekallekrein, HMW kininogen, factor X and factor XII. Thus, the isolated factors II, VII, and X need to be pure and not have any detectable amounts of factors from the contact activation system. Said factors, e.g. human factors II, VII and X, i.e. K-vitamin dependent factors, may also be recombinant and added to e.g. avian or bovine plasma.

Alternative method to clear away contact factors from plasma are methods where the coagulation factors needed for a control material for prothrombin time, i.e. prothrombin, factor V, VII and X and eventually fibrinogen (depending on prothrombin time method variant to be controlled) is to precipitate the K-vitamin dependent factors in human plasma from healthy donors utilizing a barium salt, for example barium sulphate and add purified factor V and fibrinogen (if needed). Concentrates of K-vitamin dependent factors derived from human plasma are commercially available for intravenous treatment of patients, for example Ocplex® (Octapharma) and Confidex® (from CSL Behring).

In one particular embodiment, the plasma is human citrated plasma, the calcium level is physiological, factor XII is depleted to about 98, 99 or even 99.5% or above compared to undepleted plasma and the stability is about 3 months in RT and about 1.5 years in 2-8°C.

In further embodiments, the stable solution of the invention is an aqueous solution.

Since said solution of the invention is stable there is no need for lyophilization for storage. Thus, it may not need to be lyophilized. Accordingly, still further embodiments are wherein the solution is an aqueous solution with the proviso it has not been lyophilized.

However, out of convenience, the stable solution according to the invention may still be lyophilized, albeit not necessary for stabilization purpose of the solution. If the solution is lyophilized, the lyophilized solution is reconstituted prior use. However, no further additives are needed upon use and the re-constituted stable solution is stable as described herein. Sometimes a lyophilized solution is more convenient for e.g. transportation.

Despite being held in solution or being lyophilized, reconstituted or just kept as a solution without lyophilization, the stability of the stable solution of the invention is >4h as given herein.

The solution described herein may be lyophilized for storage and reconstituted in a suitable carrier prior to use. Any suitable lyophilization method (e.g. spray drying, cake drying) and/or reconstitution techniques can be employed.

It will be appreciated by those skilled in the art that lyophilization and the subsequent reconstitution before use can lead to varying degrees of activity loss.

The lyophilized (freeze dried) composition may lose its activity upon re-hydration. However, normally it loses no more than about 20%, or no more than about 25%, or no more than about 30%, or no more than about 35%, or no more than about 40%, or no more than about 45%, or no more than about 50% of its activity (prior to lyophilization) when re-hydrated compared to prior lyophilization.

*Use of the stable solution*

Further aspects of the invention provide uses of the stable solution of the invention as control material for coagulation analysis. The stable solution of the invention with an increased, i.e. >4h, stability at 2-8°C and also stabile for >4h in RT, is particularly suitable for use as control material for blood coagulation analysis due to its increased stability. Thus, less reconstitutions of lyophilized material, less human errors made due to such dilution steps, less variability between tests and more consistent and reliable results will be achieved. Particularly for embodiments wherein washed erythrocytes are added to the stable solution of the invention less recalculations via algorithms and standard curves on e.g. a special coding chip normally used for the control sample in the art (e.g. CoaguChek XS) are needed. Indeed, again, this elimination to use only one coding chip will reduce and eliminate errors and mistakes. Thus, more reliable results will be achieved aiding at guiding further medication to the patient. The patient will get correct dose of blood medicine which in turn will increase life quality of the patient as well as decrease hospitality time and use of trained staff thereto thereby reducing total costs of the society. Normally, the HEPES-buffer used is 25 mM HEPES pH 7.4 with 33 mmol/L calcium.

*Common screening test for coagulation- APTT, ACT and PT.*

*Activated partial thromboplastin time (APTT)*

The method activated partial thromboplastin time (APTT) was developed to solve problems with the older screening method for coagulation factor deficiencies, the partial thromboplastin time. The cephalin (brain extract without tissue factor, i.e. "partial thromboplastin") used in different laboratories varied in potency and, most important, plasma was activated to various degree by contact with foreign surfaces. Proctor and Rapaport (1) bypassed the problem by maximally activating the citrated plasma with a suspension of kaolin powder for 3 minutes at +37°C followed by recalcification and determination of the clotting time. Other materials used as contact activators are silica and ellagic acid. The preincubation step initiates contact activation in which factors XII and XI are activated by prekallikrein and high-molecular weight kiningen, facilitated by the phospholipids. By adding adsorbed plasma to the APTT one could detect specific deficiencies.

The analysis procedure consists of two steps;

> Step 1: Citrated plasma + phospholipids + contact activator $\rightarrow$ activation of fXI and XII
> Step 2 Recalcification $\rightarrow$ Fibrin clot

APTT is thus dependent on the intrinsic pathway of the coagulation system, i.e. factors VIII, IX, XI, XII, prekallikrein and high-molecular weight kiningen and factors of the common pathway, i.e. fibrinogen, prothrombin, factor V and X. Factors VII and XIII have no influence at all on the APTT. APTT is used as screening test for deficiencies in coagulation factors, to monitor therapy with unfractionated heparins and to detect lupus anticoagulant.

It is not possible to standardize the APTT and introduce common units because different reagents and instruments vary widely in responsiveness to factor levels and heparins. Some reagents are more sensitive for factor deficiencies and other less but more sensitive for lupus anticoagulant. The reference interval for healthy controls must be determined locally. Causes of falsely prolonged APTT are contamination of heparin used for flushing indwelling catheters, too high citrate concentration in the sample due to under-filling and partially clotted samples due to insufficient mixing.

APTT is most sensitive for factor deficiencies at the start of the intrinsic system. Mild factor deficiencies (> 25 %) in factor VIII or IX usually go undetected, but for some reagents even a factor concentration of only 10% might go undetected! For example, some years ago the correct diagnosis of hemophilia in a young boy was delayed more than two months following a bleeding in the knee joint, partly due to an insensitive reagent and an APTT within reference range. Most reagents are less sensitive to factor IX deficiencies than to other factor deficiencies. It must be emphasized that many patients with mild, and in worst case even moderate, bleeding disorders will have a normal APTT.

The responsiveness to heparin varies at least two-fold between reagents and thus results in a prolonging of the APTT of 1.5-2.5 or 2-3 -fold compared to the control value. However, the dose of heparin will vary widely depending on the local reagent-instrument combination. APTT may vary considerably even between instruments of same make and between reagent lots. An important cause of falsely short APTT is leakage of platelet factor 4 from the platelets in the blood sample neutralising the heparin, thus it is important to separate the plasma shortly after blood sampling (within one hour).

However, intravenous treatment of venous thromboembolism with un-fractionated heparin (UFH) has diminished very much in recent years and has been substituted with low molecular weight heparins (LMH) given weight-adjusted subcutaneously without laboratory monitoring with APTT. LMH treatment prolongs the APTT, at prophylactic doses only a few seconds but at doses used for treatment of venous thrombosis the APTT usually will be some seconds above the upper limit of the reference interval. Treatment with warfarin also prolongs APTT, at INR 2-3 commonly 5-15 sec above the upper limit of the reference interval, but when heavily overdosed the APTT may become prolonged to >180 sec.

*Activated clotting time (ACT)*

Hattersley adjusted the APTT test for samples of fresh whole blood using diatomite as activating reagent. He named the test activated coagulation time (ACT) (2). Nowadays kaolin and celite are the two most used contact activators, sometimes used in combination. Celite is more responsive to aprotinin than is kaolin (3). ACT has a wider dose-range response than APTT so it is used for monitoring high-dose UFH therapy. In contrast to APTT, ACT is still one of the most frequently performed coagulation analyses and the number of tests are increasing, at point-of-care to control treatment with intravenous UFH in open-heart surgery and in percutaneous coronary intervention. Commonly a heparin dosage aims at a coagulation time of >420-480 sec in order to avoid clotting when connecting the patient to the extra-corporeal circuit. Depending on reagents and instrument, the ACT for a plasma sample from a patient on high dose heparin may vary considerably, even between duplicate samples. The variation is worse than for APTT (4). Some manufacturers have introduced algorithms in the software of the instrument converting the measured clotting time to become equal to the old manual method at the decision limit for open heart surgery in order to improve customer acceptance.

*Prothrombin time (PT, PT-INR, PK-INR)*

PT is used for three purposes; to monitor oral anticoagulant therapy with coumarins, to asses liver function in severe liver disease, and to screen for deficiencies in the extrinsic and common pathways. Two different assay types are used. The assay type invented by Quick in the 1930s (5, 6) is characterized by the use of undiluted citrated plasma mixed with equal volumes of thromboplastin reagent (= plain thromboplastin) and calcium chloride solution or two volumes of a mixture of thromboplastin and calcium chloride. Thromboplastin is an extract containing tissue factor and phospholipids, usually from rabbit brain or human placenta - sources rich in tissue factor. In recent years, recombinant tissue factor has been introduced. The Quick PT result depends on the activity of the vitamin-K-dependent factors II, VII, X and of factor V and fibrinogen. Factor V is unstable and thus blood samples have to be analysed within one hour after blood sampling or plasma has to be separated and frozen.

Schematically, the analysis procedure is as follows;

Citrated plasma + thromboplastin + Ca2+ → Fibrin clot

A more specific assay type was described by Owren almost two decades later (7, 8). It is characterized by mixing the patient sample with a reagent containing thromboplastin, factor V, fibrinogen and calcium chloride (=combined thromboplastin). Due to the addition of factor V and fibrinogen, often in the form of factors II, VII and X depleted bovine plasma, this assay is more specific for the coagulation factors II, VII and X. If PT is analyzed by the Owren method samples from patients on warfarin may be stored at room temperature for 48 hours.

PT in samples from patients on oral anticoagulation depends mostly on prothrombin and factor VII.

In brief, the analysis procedure for the Owren PT is as follows;

Citrated plasma prediluted in buffer 1/7 + bovine depleted plasma + rabbit thromboplastin + Ca2+ → Fibrin clot

The Owren method has been modified and in the Owren-type PT assay procedure currently performed in the Nordic and Baltic countries, there is an initial plasma sample dilution (1 part +6 parts) with buffer containing citrate before addition of the combined reagent (2 parts) to the diluted sample (1 part), giving a final sample dilution of 1/21. This can be contrasted with the original Quick PT assay which has a final sample dilution of 1/3. In Quick PT assays modified for point-of-care utilising lyophilised thromboplastin dissolved by the native blood sample, the patient plasma is not diluted at all, for example in the CoaguChek instrument (Roche Diagnostics, Basel, Switzerland).

The advantage of the current Owren method is that a minimal amount of sample is needed for the PT test, and the test is therefore suitable for automated laboratory analysis of paediatric and capillary blood samples. The dilution also reduces interference, which is desirable particularly when lupus anticoagulant or other inhibitors such as direct thrombin inhibitors are present (9, 10). With the Quick method, and in particular if applied to point-of-care instruments, lupus anticoagulant may increase the INR-value considerably and patients are at risk for under-treatment and thrombosis.

With PT the primary result is clotting time in seconds, for normal plasma with the Quick method about 12 seconds, with the Owren method about 20 seconds. The fibrin formation is usually detected by increase in absorbance by turbidometry or nephelometry, alternatively by detection of the increase in viscosity by mechanical devices. Different thromboplastins exhibit variable sensitivities to factor deficiencies. Mild factor deficiencies (>40%) may go undetected. The most common causes of spontaneously prolonged PT are liver disease, hereditary factor VII deficiency (usually mild without bleeding symptoms) and insufficient vitamin K intake. Therapeutic doses of UFH prolong the PT by a few seconds unless the reagent contains a heparin neutralizer. Capillary drawn citrated whole blood is conveniently analyzed by the Owren method thanks to the low blood volume required, but very high or low hematocrit will cause erroneous results unless the instrument measures and compensates for the hematocrit, done for example by the point-of-care instrument Simple Simon® (Zafena AB, Borensberg, Sweden). For many years, the PT results were expressed in seconds, prothrombin index, prothrombin activity, or prothrombin ratio (11). Depending on reagent, the results and consequently the mean doses of coumarins varied widely between hospitals. In order to standardize the PT the International Normalized Ratios (INR) was adopted by the World Health Organisation 1983 (12). INR= (PT/MNPT) ISI.

PT is the prothrombin time in seconds, MNPT is the geometric mean of PT of plasma samples from at least 20 normal subjects and ISI is the International Sensitivity Index of the thromboplastin.

The idea of ISI is to convert the ratio obtained with local working method to the ratio which would have been obtained with the first international reference preparation (IRP) of thromboplastin designated 67/40, using the manual tilt tube technique. The ISI of the working method is determined by the split-sample PT testing against the reference method and an IRP. Fresh plasmas from at least 20 normal subjects and 60 patients on stable oral anticoagulation must be tested by both methods. The ISI is the slope of the orthogonal regression line of the reference method log-PT on the working method log-PT. The original stock of the IRP 67/40 has been exhausted and chains of secondary IRPs have been assigned ISI-values in a hierarchical mode. Depending on which secondary IRP is used the results will deviate remarkably, due to errors introduced in each assignment and minute changes of properties during storage of the IRPs. The WHO protocol is cumbersome, the stocks of IRPs are limited and expensive and calibration has to be done in each laboratory some times each year (when changing reagent lots, instruments or after service of instrument). Very few labs uses the WHO protocol but relies instead on ISI-values provided by manufacturers. However, ISI values assigned to classes of instruments or models are not necessarily valid for specific instruments. Due to the limitations of calibration procedure described above there is an increasing interest in using plasma calibrators with assigned INR-values. However, different IRPs, analytical methods and production methods of calibrators may result in significant different assigned INR values. Two examples of additional unresolved questions are how many calibrators are needed and matrix effects of calibrators of calibrators versus fresh patient plasmas.

In Sweden and Norway, where the Owren type PT is used, an alternative calibration procedure was introduced in 1999 (13). The method utilized dilutions of normal plasma instead of IRP and the percent of normal activity (PT%) was converted to INR by using an equation derived from a regression analysis based on split-sample analysis of plasma samples from normal subjects and patients on oral anticoagulation using the Owren method and the manual tilt-tube technique and an IRP (INR = (1/PT% + 0.018) / 0.028). For example 100%= INR 1, 25%=INR 2.07, 20%=INR 2.43, 10%=4.21. Using this algorithm any dilution of normal plasmas has an INR value, valid for ever, which is used to assign INR-values to lyophilized calibrators. A reference thromboplastin (IRP) is therefore not required any longer. At the local laboratory level only two calibrators are needed, one in the normal range and one with INR in the therapeutic range. A three-year follow-up in Sweden where several laboratories had participated in an external quality control program, reported that the intra- and inter-laboratory variations had become markedly improved with this local INR calibration (14). Despite different modes of calibration used for the Owren-PT in Denmark, Finland and Iceland, the inter-laboratory variation in the Nordic countries is very small in an international perspective (15).

In order to obtain correct results with the Owren type PT is it important to mix the blood sample to ensure anticoagulation and homogenous plasma, to have samples and reagents equilibrated to correct temperature before analysis, to dissolve the reagent the required time before analysis (to avoid changes in ISI and/or MNPT during the working day), to perform a correct calibration, to run control samples and adhere to rules for QC.

Despite PT has been used for more than 60 years in control of oral anticoagulation, there is still need for improvement of reagents and calibration procedures. In countries using the Quick-method the between laboratory variation is considerably and for a patient sample clinically significantly different result may be obtained due to reagent properties. A significant improvement would be achieved if countries still using the Quick PT-method changed to the more specific Owren PT-method.

*Methods of producing a stable solution*

Further aspects of the invention are methods of producing a stable solution, the method comprising

a) isolating plasma

b) depleting the plasma of factor XII, and

c) adjusting levels of free calcium to ≥0,5 mmol/L, physiological levels or above.

A further aspect of the present invention is to provide a method for producing a stable solution of the invention, wherein said method comprises the steps of a) adding isolated human factor II, VII, and X to isolated plasma, and b) adjusting levels of free calcium to ≥0,5 mmol/L.

A further aspect of the invention is to provide a method for producing a stable solution, the method comprising the steps of a) adding isolated human factor II, VII, and X to isolated plasma, and b) adjusting levels of free calcium to ≥0.5 mmol/L.

Still, a further method is to a) isolate human factor II, VII, X, b) adding non-human plasma depleted of at least one contact-activation factor, such as e.g. prekallekrein, HMW kiniogen, Factor V, Factor XI or factor XII, c) adjusting .levels of free calcium to ≥0,5 mmol/L, physiological levels or above.

As described herein, the isolated plasma may be mammalian, such as human plasma, or non-human mammalian plasma, such as rabbit or bovine. It may also be non-mammalian plasma such as avian plasma. As mentioned herein, if avian plasma is used, no further depletion of at least on contact-activating factor is needed.

If mammalian non-human plasma is used, said methods as described herein further comprise the step of depleting the mammalian non-human plasma of at least one contact-activating factor.

Since calcium levels of the stable solution is physiological or above, a step d) being optional, is wherein the calcium levels are adjusted to physiological depending on if a calcium chelator or not was added upon withdrawal of the blood. Normally, however, a calcium chelator is added and the subsequent optional step of adjusting the calcium levels to physiological or above is mandatory. The optional step is due to the source of blood for isolated plasma. If a normal blood donor is withdrawn of blood, the calcium levels needs to be adjusted to physiological level or above. If the blood donor is deficient in factor XII, i.e. deficient in activity or protein expression per see, no additional calcium needs to be added since the calcium levels will be physiological or above since no chelator or anti-coagulant needs to be added upon withdrawal of the blood. Thus, in the method above for the preparation of said stable solution, calcium levels needs to be checked for normal levels or above and adjusted to the same if so needed, i.e. if the calcium levels are below normal. Ionized Calcium levels in blood or blood plasma may be measured by most blood gas analyzers, for example Radiometer ABL 720, or the Kone clinical chemistry analyzer. See http://www.radiometer.com/ and http://www.thermofisher.com/global/en/products/home.asp, respectively. See e.g. Laurells Klinisk kemi. Ed Nilsson-Ehle P. Studentlitteratur 2003. +Brown EM, MacLeod J. Extracellular calcium sensing and extracellular calcium signalling. Physiol Rev 2001;81:239-97+ Larsson L, Magnusson P. Ionized calcium or corrected total calcium? J Bone Miner Res 2003;18:1554-5.

Plasma may be isolated by normal venipuncture, in e.g. citrate (citric acid buffer), isocitrate or oxalat buffer as chelating agent as is normally standard, or similar. Citric acid buffer is normally added at about 1/10 volume of about 0.105, 0.11 or 0.13 mol/L final concentration, neutral or slightly acidic pH, e.g. about pH 6-7.4, i.e. in large excess to free calcium in the plasma.

If the donor has an inherited factor XII deficiency, blood is withdrawn without any calcium chelating agent added. The blood is then collected in e.g. plastic tubes without any further additives such as the citrate (citric acid) or isocitrate buffer, e.g. using Monovette, Sarstedt, using aspiration or vacuum principle of collection. The blood is then centrifuged and the buffy coat and the uppermost part of the erythrocytes are removed as described herein. Following a second centrifugation, plasma is saved and the pellet discarded.

If needed, depletion of factor XII is then performed. How to deplete plasma of factor XII is known in the art and exemplified further herein by e.g. immune methods, depletion using a metal oxide, such as e.g. titanium oxide, and other methods. Depletion of at least one further contact activating factor, if needed, may also be done using immune methods and similar known to the skilled artisan.

Calcium levels are further measured and calcium added to the blood plasma to adjust the calcium level to physiological or above.

Further, the factor XII-depleted plasma may then be kept as a stable solution according to the invention or lyophilized. If lyophilized, a reconstitution before use is needed. However, lyophilization is not needed for stabilization purposes. The solution may then, after reconstitution be kept as a stable solution.

The method for preparation of said stable solution may then further comprise a step for depletion of platelets in the plasma. Platelets may be depleted by centrifugation at about 1-80-200xg followed by filtration through a filter with a pore size not more than 0.25μm, as described herein.

The method according to the invention for preparation of the stable solution may then further comprise adding of washed erythrocytes. Washing of erythrocytes are known in the art and further described herein. Washing may be done by centrifugation at 1000xg, re- suspension physiological saline in equal amount and repeated centrifugation.

Further embodiments are thus wherein the plasma is human plasma.

Still further embodiments are wherein the added washed erythrocytes are human erythrocytes.

Further aspects of the invention are wherein the stable solution according to the invention is obtainable by the methods described herein.

*Methods for assessing coagulation system status*

Further aspects of the invention encompass methods of assessing coagulation system status in a subject, the method comprising

a) providing a stable solution according to the invention,

b) providing a blood sample from a subject,

c) measuring status of the coagulation system of said subject,

d) analyzing status of the coagulation system of said patient,

e) compare status of the coagulation of said subject to a positive and/or negative standard, thereby assessing the coagulation system status in said patient.

Still a further aspect of the invention provides a method for assessing coagulation system status in a subject, the method comprising the steps of a) measuring status of the coagulation system of said subject in a blood sample from said subject, and b) analyzing status of the coagulation system of said subject in relation to a stable solution of the invention, thereby assessing the coagulation system status in said subject.

Said method is with the provision that no further calcium is added to the solution immediately before use, as is normally the case in blood coagulation assays. Thus, the stable solution is a solution with calcium levels being physiological and with the proviso that no calcium is added to the stable solution by the person/personal running the point-of-care instrument analyzing fresh blood samples and the control material.

The measuring of the status of the coagulation system may be done by different tests known in the art to assess the function of the coagulation system. The stable solution of the present invention may then be used as a control material in the assay.

Examples of assays/tests are e.g. PT (prothrombin time, used also to determine INR), mixing test (whether an abnormality of a patient is corrected if the patient's plasma is mixed with normal plasma), coagulation factor assays, thromboelastography (TEG or Sonoclot).

The contact activation pathway is initiated by activation of *contact factors* of plasma, and can be measured by a aPTT test.

The tissue factor pathway is initiated by release of *tissue factor* (a specific cellular lipoprotein), and can be measured by a PT test. PT results are often reported as ratio, i.e. INR (International Normalised Ratio) value.

After measuring, an analysis is performed of each sample and the value given to the sample in the measuring step is analyzed.

Further, the subsequent comparison related to a positive and/or negative control included in the assay may be done.

The present stable solution of the invention may be used herewith. The stable solution of the invention may also be used as a mixing component if the assay is performed as a mixing assay.

For such a mixing assay K-vitamin dependent factors must also be depleted from one mixing solution to be mixed with one solution with normal content of these factors. Depletion of the K-vitamin dependent factors may be done by use of absorption to barium salts or by immunodepletion. Prothrombin complex activity in per cent may be converted to INR by use of a published algorithm (Lindahl TL, et al. INR calibration of Owren-type prothrombin time based on the relationship between PT% and INR utilizing normal plasma samples. Thromb Haemost 2004;91:1224-31).

Further embodiments are wherein the subject is a human subject.

The status of the coagulation system may be measured by an assay selected from the group consisting of PT (prothrombin time), mixing test, coagulation factor assays, thromboelastography (TEG or Sonoclot), and euglobulin lysis time (ELT).

The method may further comprise wherein the blood sample from a subject is mixed with the stable solution according to the invention before measuring and analyzing the status of the coagulation system.

*Kits*

Further aspects if the invention encompasses a kit comprising the stable solution according to the invention.

The stable solution may be provided in lyophilized form, if convenient. However, this is not needed for stability purpose of said stable solution of the invention.

In some embodiments, a kit includes instructional materials disclosing, for example, means of use of the stable solution of the invention as control material for blood coagulation analysis/assays or means of use for a particular reagent. The instructional materials may be written, in an electronic form (e.g., computer diskette or compact disk) or may be visual (e.g., video files). The kits may also include additional components to facilitate the particular application for which the kit is designed. Thus, for example, the kit can include buffers and other reagents routinely used for the practice of a particular disclosed method. Such kits and appropriate contents are well known to those of skill in the art.

The kit may further comprise, in an amount sufficient for at least one assay, the stable solution according to the invention as a separately packaged reagent.

Instructions for use of the packaged reagent are also typically included. Such instructions typically include a tangible expression describing reagent concentrations and/or at least one assay method parameter such as the relative amounts of reagent and sample to be mixed, maintenance time periods for reagent/sample admixtures, temperature, buffer conditions and the like.

Thus, further embodiments are wherein the kit further comprises instruction for its use as control material for coagulation analysis.

Still even further embodiments are wherein the kit further comprises instruction for its use in a method according to the invention.

Certain kit embodiments can include a carrier means, such as a box, a bag, a satchel, plastic carton (such as moulded plastic or other clear packaging), wrapper (such as, a sealed or sealable plastic, paper, or metallic wrapper), or other container.

In some examples, kit components will be enclosed in a single packaging unit, such as a box or other container, which packaging unit may have compartments into which one or more components of the kit can be placed. In other examples, a kit includes a one or more containers, for instance vials, tubes, and the like that can retain, for example, one or more blood samples to be tested.

Other kit embodiments include, for instance, syringes, cotton swabs, or latex gloves, which may be useful for handling, collecting and/or processing a blood sample. Kits may also optionally contain implements useful for moving a blood sample from one location to another, including, for example, droppers, syringes, and the like. Still other kit embodiments may include disposal means for discarding used or no longer needed items (such as subject samples, etc.). Such disposal means can include, without limitation, containers that are capable of containing leakage from discarded materials, such as plastic, metal or other impermeable bags, boxes or containers.

All references herein are incorporated by reference.

Non-limiting examples which embody certain aspects of the invention will now be described.

EXAMPLES

*Example 1 - Purification of factor XII deficient blood from donors with factor XII deficiency and measurement of protrombin time*

*Collection of time*

Blood was collected from two donors with inherited factor XII deficiency (i.e. factor XII below detection limit as measured by clotting time using immunodepleted plasma) by venipuncture, blood collected in plastic tubes without additives (Monovette, Sarstedt) (no anticoagulant additives). The blood was centrifuged at 1 80xg for 20 minutes. The buffy coat and the uppermost part of the erythrocytes were removed, a second centrifugation step at 1 000xg for 5 min followed. The plasma was saved and the pellet discarded.

*Washing of red blood cells*

Blood was collected from health donors by venipuncture of an anticubital vein and collected in evacuated tubes containing 1/10 volume of sodium citrate (0.13mol/l). The tubes were centrifuged at 1 80xg for 20 minutes, the platelet-rich plasama and the uppermost part of red blood cells were discarded. An equal volume of Owren's buffer was added. The red blood cell pellet was resuspended, followed by a centrifugation at 1 000xg for 5 minutes. The buffer phase was discarded, an equal volume of a physiological saline was added and the centrifugation step was repeated. The blood cells were mixed before addition to the factor XII-depleted plasma.

*Measurement of prothrombin time utilizing the point-of-care instrument CoaguChek XS.*

For plasma the instructions for control material from the manufacturer of the instrument were followed, i.e. the code-chip for control material was inserted in the instrument as instructed.

Different Lots of reagent has been used, for example 183 and for control material provided by Roche code chip 028, lot 170. For CoaguCheck sticks eg. Lot 20165732.

*Results*

Results are seen in Table 1.

- Undiluted Factor XII-deficient plasma: INR= 1.0 (expected result for healthy donors; INR $1\pm0.2$). This factor XII-deficient plasma was obtained from volunteering patients with known hereditary, total (below detection limit utlizing the one stage clotting method on ACL Top from II (Milan, Italy)) deficiency of factor XII.

- Factor XII-deficient plasma pre-diluted 1+1in HEPES-buffer; INR= 1.9;1.9 and1.8 (n=3)

- Using the new material following the instruction for capillary, fresh blood obtained from patients on oral anticoagulation or healthy donors, i.e. only using the coding chip for fresh, undiluted blood.

- Undiluted factor XII-depleted plasma was mixed 1+1 volumes with washed red blood cells and factor XII-deficient plasma and then measured.

Results: INR= 1.1;1.2;1.2 expected result INR $1\pm0.2$)

- As above but the factor XII-depleted plasma was diluted with an equal volume of HEPES-buffer before mixing with the washed red blood cells.

Results: INR= 2.4;2.5;2.5)

The factor XII-deficient plasma was exchanged with factor XII immuno-depleted plasma, Cryocheck™ (Precision Biologic, Cat No # FDP-12-10, Lot #D12-12) shipped frozen. This plasma was mixed with an equal volume of 25 mM CaCl2. Measured on Coaguchek XS as a control sample according to instructions from Roche.

Results: INR= 1.5;1.5;1.5 (expected value INR= 1.4 $\pm0.2$).

Factor XII-deficient plasma (from patient) was mixed with 3 volumes of HEPES-buffer, INR= 1.9;1.9;1.9 (expected value 2.0$\pm$0.2) (Undiluted INR=1.0, as before) Measured after storage at +22C for 16 hours.

Results: INR=2.0;1 .9,1.9 , i.e. no difference at all!

The deficient plasma was diluted 1+3 in 25 mM HEPES-buffer with 33 mmol/L $CaCl_2$ with Na-azide, final concentration 5 mmol/L.

**Table 1 Stability of plasma from a new patient deficient in factor XII**

|  | Day=0 | 1 | 2 | 7 |
|---|---|---|---|---|
| INR | 1.9 | 1.8 | 2.0 | 2.4 |

*Example 2 - Stability tests*

A. Factor XII absorbed to <1%.
Plasma collected from a normal donor is diluted in HEPES-buffert (4.86g HEPES to 1 L water) using 18.9 mL HEPES-buffert to 10 mL plasma. To adjust the calcium concentration to physiological 1.33 mL of a calcium chloride stock solution (0.5 mol/L).
150 microliter of a stock solution of azide (1 mol/L) is added to prevent bacterial growth.

Results stability are seen in table 2.

**Table 2 - Stability at day 3, 5, 8, and 12**

|  |  |  |  | *3 days* | *5 days* | *8dygn* | *12 days* |
|---|---|---|---|---|---|---|---|
| **0** | 2h | 4h | 24h | 48h | 120h | 192h | 288h |
| **INR** | 1,6 | 1,7 | 1,8 | 1,8 | 1,8 | 1,9 | 1,9 |
|  |  |  |  |  |  |  |  |

When using this way of producing the stable solution, the INR might vary slightly the first hour. However, after 24h it is stable. The variation INR-value due to the apparatus is about 0,1 INR. The solution was kept in room temperature.
B. In this example plasma from a Factor XII-deficient subject is used. The deficiency leaves less <1% of Factor XII in the blood of said subject. No citrate is added to the plasma. No azide is added to the plasma. Results are seen in Table 3.

**Table 3 - stability at 24h, 48h, 3 days and 4 days.**

| Fresh made | 24 h | 48 h | 3 days | 4 days |
|---|---|---|---|---|
| 1,2 | 1,3 | 1,3 | 1,3 | 1,3 |

C. In the below example has the Factor XII-plasma from B been diluted in HEPES-buffer (including 0.13 mol/L sodium chloride) to get a higher INR-value Data from stability measurements are seen in Table 4.

**.Table 4 - stability at 24hm 48h, 3 days, 4 days, and a week.**

| Fresh made | 24h | 48 h | 3 days | 4 days | 1 week |
|---|---|---|---|---|---|
| 2,1 | 2,2 | 2,3 | 2,5 | 2,5 | 2,9 |

In this case, the stability was only one day.

*Experiment No 3 - Production of plasma deficient in coagulation factor XI and/or Factor XII*

Antibodies directed towards factor XI (polyclonal chicken antibody directed towards human factor XII) and XII (goat anti-human factor XII purified IgG) was coupled to Novarose™ Act[High] 100/40 gel from Innovata (Stockholm, Sweden) respectively according to instructions from manufacturer.
Citrated human plasma was applied on top of the gel, fractions were collected. The absorbance at 280 nm was measured. Fractions containing most proteins (highest absorbance) were analyzed using PT (Owren method). The fractions with PT-INR <3 were saved and stored at -70°C. Some of the fractions were applied to the column with the other immobilized antibody as well, thus making double deficient plasma.
The deficient plasma was diluted 1+3 in a low ionic strength HEPES- buffer (25 mmol/L, pH 7.4) containing 8.8 mmol/L $CaCl_2$. The low ionic strength buffer was used in order to be able to use the Coaguchek XS instrument with code chip and test strips with intended use for native human capillary blood (Impedance measurement is used in the instrument to check if sample is blood or plasma, if not as expected the instrument gives no result but an error message. The intention here was to use the same procedure as for whole blood, hence the use of this low ionic strength buffer.
Considering dilution effects we expected a result slightly above INR=2 for plasma deficient in one factor (if no dilution in production of the deficient plasma 2.0 and 3.3 would be expected), about 3.5 for double deficient plasma. Obtained values below Table 5 and 6 are thus as expected:

**Table 5 - stability measurements over 24h for factor XI, XII and double deleted plasma.**

| Antibody used | INR at time zero | INR at 24 h | INR at 8 days |
|---|---|---|---|
| FXI | 2.3 | 2.4 | 4.2 |
| FXII | 2.4 | 3.1 | Nd |
| FXI and FXII (1 vol)* | 3.4 | 3.9 | Nd |

(continued)

| Antibody used | INR at time zero | INR at 24 h | INR at 8 days |
|---|---|---|---|
| FXI and XII (2 vol)** | 2.1 | 2.7 | Nd |
| *) deficient plasma mixed with the low ionic strength buffer 1 +3 **) 2+3. | | | |

The absorption of FXII with the goat antibody seemed to leave traces of FXII in the breakthrough fractions collected. FXI absorption was more efficient in this particular case with promising stability although not for 8 days.

**Table 6 - New batch of plasma**

| Antibody used | INR at time zero | INR day 1 | INR day 2 | INR day 7 |
|---|---|---|---|---|
| FXI | 6.6 | 2.8 | 2.7 | 1.8 |

The peculiar change first 24 hours is not known why and did not occur in the previous experiment above. However, what is important is the stability from day 1 and onwards.

*Experiment 4 - Production of FXII-deficient plasma using absorption to different negatively charged materials and comparison of stability of deficient plasma obtained by such absorption*

*Blood plasma preparation*

Fresh blood was drawn from healthy volunteers and collected in 4.5 mL Monovette sampling tubes (Sarstedt, Germany) with citrate. To obtain platelet-rich plasma (PRP), the fresh blood was centrifuged at 140g for 20 min. After centrifugation, the platelet-rich supernatant was transferred to a new plastic tube. Platelet-free plasma (PFP) was produced by centrifugation of blood at 2500g for 15 min and filtration through a 0.20 micrometer Minisart membrane filter (Sartorius, Germany).

*Factor XIIa activity*

Factor XIIa activity was measured using factor XIIa fluorogenic substrate Boc-Gln-Gly-Arg-AMC (see [1]) in citrated plasma. Plasma was incubated in either titanium dioxide or kaolin powder for 5 minutes, using a ratio of 0,5 g powder per milliliter plasma. The plasma samples were stored in a refrigerator and sample aliquots were frozen at 1 hour, 1 day, 2 days and 4 days. Thereafter all samples were thawed and assessed with 250 micromolar factor XIIa activity substrate. Fluorescence measurements were carried out in a Fluoroskan 96-well plate reader, using excitation wavelength 390 nm and emission 460 nm.

Results were calculated as the max factor XIIa substrate cleavage rate and are presented as mean+SD in Fig. 2. From the results a higher level of factor XIIa activity is found in the kaolin treated plasma samples. This level is however decreasing with time ($p < 0.05$ comparing Day 0 and Day 4 using paired samples t-test). Results are seen in Figure 2.

*Example 5 - Endogenous thrombin potential (ETP)*

Endogenous thrombin potential (ETP) was measured with a calibrated thrombin generation assay (Thrombinoscope) utilizing a fluorogenic substrate for thrombin (see [2]). ETP can according to H.C. Hemker be used to "assess the combined effect of all the factors that may influence thrombin generation in a given plasma sample" [3], i.e. what is relevant for a control material for prothrombin time.

Citrated platelet-free plasma (see blood plasma preparation paragraph) isolated from three different individual was incubated in either titanium dioxide or kaolin powder for 5 minutes, using a ratio of 0,5 g powder per milliliter plasma. The plasma samples were stored in the refrigerator and sample aliquots were frozen at 1 hour, 1 day, 2 days and 4 days. Thereafter all samples were thawed and assessed with the thrombin generation method described by Hemker et al. [2] Thombin generation was calculated as ETP using the Thrombinoscope software package (Thrombinoscope BV, The Netherlands). Note that plasma from kaolin incubation did only produce flat thrombin generation curves, which gives an ETP of zero. In conclusion, kaolin absorption is not an option for production of control material. See Figure 3.

Figure 3 shows thrombin generation calculated as endogenous thrombin potential (ETP) presented as mean + SD after 0, 1, 2 and 4 days. Data represents incubation with titanium dioxide during 5 minutes (striped) and 60 minutes (solid black), and incubation with kaolin during 5 minutes (white with black dots) and 60 minutes (black with white dots) minutes respectively.

*Example 6 - Prothombin time (PT) measured with Coaguchek (Roche)*

Citrated platelet-free plasma (see blood plasma preparation paragraph) isolated from three different individuals was incubated in either titanium dioxide or kaolin powder for 5 minutes, using a ratio of 0,5 g powder per milliliter plasma. The plasma samples were then stored in the refrigerator four days. In the PT measurement 10 microliter plasma was mixed with 30 microliter the low ionic strength buffer containing calcium. Thereafter the prepared plasma samples were measured in the Coaguchek and the INR or error code recorded. Plasma incubated in kaolin did only generate error codes (3 of 3 donors) in the PT measurements, whereas plasma samples incubated in titanium dioxide could all be measured and resulted in INR values (3 of 3). Results are seen in Table 7.

**Table 7. INR values or error codes from prothombin time measurements using a Coaguchek(Roche)**

| Material | Donor | INR | Error code |
|----------|-------|-----|------------|
| Titanium dioxide | 1 | 6,7 | - |
| Kaolin | 1 | - | 6* |
| Titanium dioxide | 2 | 7,8 | - |
| Kaolin | 2 | - | 6* |
| Titanium dioxide | 3 | 5,5 | - |
| Kaolin | 3 | - | 6* |
| *Error code 6 indicates "Measurement error" according to the Coaguchek XS Plus manual. | | | |

*Example 7 - Material dependent sequestering of activated coagulation factors*

Using an imaging system previously described by Faxälv et al. to study coagulation on material surfaces (see [4]), coagulation times at the surface of Fe, Al, Ti and glass material pieces were measured. Ti, Fe and Al were obtained from Sigma-Aldrich, Sweden. Coagulation times at the material surfaces were measured using the materials directly or after 60 minutes incubation in citrated platelet free-plasma. Plasma incubated materials were before coagulation measurement washed in HEPES buffer containing (137 mmol/L NaCl, 2.7 mmol/L KCl, 1 mmol/L $MgCl_2$, 5.6 mmol/L glucose, 1 g/L BSA and 20 mmol/L HEPES (N-[2-hydroxyethyl]piperazine-*N'*-[2-ethanesulphonic acid]), pH 7.40). To start the imaging experiment recalcified (17.4 mM final conc.) platelet-free plasma was added to standard PMMA cuvettes containing the different materials. The analysis is described in brief; time-lapse images were captured using an EOS 400D camera (Canon, Japan) and analyzed with respect to surface coagulation time using our own software, written in MATLAB (The MathWorks, Natick, MA). Coagulation times were defined as the time required for a 40 % increase in light scattering, the light scattering before fibrin formation starts is set to 0 % and the light scattering after complete fibrin formation (coagulation) in the cuvette is set to 100 %. Coagulation times at the material surface were calculated from the time-lapse images in the image pixel column bordering closest to the surface in the image.

Coagulation times using plasma from four donors are presented as mean+SD in Fig. 4. From the results it is obvious that only Ti increases its procoagulant properties from sequestration of activated coagulation factors on the material surface.

Figure 4 shows coagulation time at the material surface using plasma from four different donors. Data is presented as mean + SD for coagulation times when measured at Fe, Al, Ti, and glass surfaces. The materials were either measured directly (solid black) or after incubation in citrated plasma during 60 minutes followed by washing in HEPES (striped).

In conclusion, this property of Ti (Titanium dioxide) may explain, at least partially, why absorption of contact activating factors utilizing titanium dioxide works to make deficient plasma, and the other materials (Fe, Al, and glass) not.

*Example 8 - Practical use of FXII-deficient plasma in External Quality Assurance Lab*

Deficient plasma was produced by absorption to titanium dioxide as described above. Dilution was done with 1 part deficient plasma +3 parts buffer with low ionic strength HEPES-buffer with $CaCl_2$ as above

Production done on day 1, is then sent by regular post at ambient temperature to office of EQUALIS (External quality assurance in laboratory medicine in Sweden) Ltd in Uppsala The stable solution is here aliquoted in labeled glass bottles and sent by regular post day 2 to the laboratories (mainly primary care health centers) all over Sweden and measured using same procedure as for fresh native capillary blood on Coaguchek XS within three days after receipt of material by just adding a few microliters of the material to the test strip. No addition or dilutions is made at the recipient lab. Data are presented in Table 8.

**Table 8 showing external quality assurance results**

| Lab | Method | Date and time | Analysis | 2010-46 |
|---|---|---|---|---|
| 1 | CoaguChek | 19/11 kl 16:25 | P-PT | 1,9 |
| 2 | CoaguChek | 2010-11-18 | P-PT | 1,8 |
| 3 | CoaguChek | 2010-11-18 11:45 | P-PT | 1,8 |
| 4 | CoaguChek | 101118 kl 13;58 | P-PT | 1,8 |
| 5 | CoaguChek | 19/11 KL.12.00 | P-PT | 2 |
| 6 instrument 1 | CoaguChek | 18/11 kl 10:35 | P-PT | 1,7 |
| 6 instrument 2 | CoaguChek | 18/11 kl 10:40 | P-PT | 1,8 |
| 7 | CoaguChek | 18/11 kl. 13:30 | P-PT | 1,8 |
| 8 | CoaguChek | 101118 kl 14.30 | P-PT | 1,8 |
| 9 | CoaguChek | 19/11 kl 09:38 | P-PT | 2 |
| 10 | CoaguChek | 19/11 kl 11:36 | P-PT | 1,9 |
| 11 | CoaguChek | 101118 kl:10:57 | P-PT | 1,8 |
| 12 instrument 1 | CoaguChek | 18/11 kl 14:00 | P-PT | 1,9 |
| 12 instrument 2 | CoaguChek | 18/11 kl 14:00 | P-PT | 1,8 |
| 13 | CoaguChek | 18/11 kl 16:20 | P-PT | 1,8 |
| Equalis | CoaguChek | 17/11 13.38 | P-PT | 1,7 |
| Equalis | CoaguChek | 18/11 kl 9.30 | P-PT | 1,7 |
| Equalis | CoaguChek | 19/11 kl 8.15 | P-PT | 1,8 |
| Equalis | CoaguChek | 22/11 kl 9.40 | P-PT | 1,8 |
| | | | mean | 1,821053 |
| | | | SD | 0,085498 |
| | | | CV% | 4,694988 |

Taking expected variation, which is a C.V. about 5% of the Coaguchek instruments, in consideration the results indicates a sufficient stability of the control material (diluted deficient plasma with ionized calcium to a level above physiological level, i.e. the stable solution of the invention). More than one batch of test strips were in use at the participating laboratories which will contribute to the imprecision.

*Example 9 - Avian plasma, which is naturally deficient of coagulation factor XII.*

It is known that some kind of animals, for examples birds (and reptiles) lack the contact activation system. Plasma from these animals may thus be used as an alternative to the absorbed human plasma or human deficient plasma (i.e. from donors with hereditary deficiency of coagulation factor XII).

In the example here below citrated (1/10 volume of 0.13 M sodium citrate in the blood sampling tube) plasma from ostrich was used. The plasma was mixed 1+3 with calcium-containing low ionic strength HEPES-buffer as above to achieve at least physiological level of ionized calcium and an elevated INR. Result from Coaguchek XS at day 0 was INR>8 and the same was recorded after 24 hours - see Table 9 and 10 below.

**Table 9 - INR at day 0, 1, 2, 5, 6, and 7**

| Ocplex addition | FV addition | Day =0 | 1 | 2 | 5 | 6 | 7 | 12 | 15 |
|---|---|---|---|---|---|---|---|---|---|
| 40 $\mu$L | No | 2.7 | 2.0 | 2.0 | 2.0 | 2.0 | 2.1 | 2.0 | 2.1 |
| 40 $\mu$L | 1 $\mu$L | 2.0 | 2.0 | 2.0 | 2.0 | 2.0 | 2.1 | n.d. | n.d |

The INR-level was thus much higher than with the same dilution of human deficient plasma, but that does not exclude use as control material.

**Table 10 - New preparation of ostrich plasma with Ocplex +/- factor V as above but with sodium azide, final concentration 5 mmol/L**

| Ocplex addition | FV addition | Day =0 | 1 | 2 | 3 | 7 | 10 |
|---|---|---|---|---|---|---|---|
| 40 $\mu$L | No | 2.7 | 1.7 | 1.7 | 1.7 | 1.7 | 1,8 |
| 40 $\mu$L | 1 $\mu$L | 1.8 | 1.7 | 1.7 | 1.7 | 1.7 | 1,7 |

40 $\mu$L purified prothrombin complex concentrate containing human coagulation factors factor II, VII, IX and X was added (K-vitamin dependent factors, Ocplex®, Octapharma, 25 U /mL) to 1 mL avian citrated plasma (final concentration of factors 0.96 U/mL).
Then 1 part (of the mixture above) was added to 3 parts of calcium-containing low ionic strength HEPES-buffer as above. The final concentration of added factors was thus about 0.25 IU/mL. The results of INR-measurements was INR=5.8 (expected value INR=2.0). With 120 $\mu$L addition of the mixture above, i.e. 40 $\mu$L purified prothrombin complex concentrate containing human coagulation factors factor II, VII, IX and X added (K-vitamin dependent factors, Ocplex®, Octapharma, 25 U /mL) to 1 mL avian citrated plasma (final conc. of factors 0.7 U/ml) INR= 2.3.
Thus the INR values were generally higher than achieved with human deficient plasma at same level of K-vitamin dependent factors. The reasons were not investigated in detail, but could at least partially be due to the properties of the avian factor V. Addition of the mixture with 40 $\mu$L Ocplex with 1 $\mu$L purified human coagulation factor V (FV) ,5.0 mg/mL lot S0517-05 MG, resulted in a lowering of INR; INR=2.0, i.e. the expected value.
For stability at room temperature, see table 11 below.

**Table 11 - Stability measurements at RT**

| Ocplex addition | Factor V addition | Day =0 | 1 | 2 |
|---|---|---|---|---|
| 40 $\mu$L | No | 2.7 | 2.0 | 2.0 |
| 40 $\mu$L | 1 $\mu$L | 2.0 | 2.0 | 2.0 |
| | | | | |

*Example 10 - purified coagulation factors as control material*

An alternative approach to use any kind of plasma made deficient in contact activation factor(s) is to purify the factors necessary for conversion of prothrombin to thrombin in the presence of tissue factor (intended as control material for methods with tissue factor present in the reagent, for example Coagucek XS test strips for prothrombin time) i.e. factor II, V, VII and X. It is very important to avoid contamination with factors from the contact activation system, i.e. prekallekrein, HMW kininogen, factor XI and factor XII.
A trial was made with only II,VII and X (i.e. Ocplex) and 40 $\mu$L added to 1 mL 25 mmol/L HEPES-buffer pH 7.4 (without calcium followed by 1+3 mixture of calcium-containing (8.8 mmol/L) low ionic strength HEPES-buffer as above (to get a free Ca concentration of about 6.6 mmol/L), INR=7.3. Obviously insufficient amounts of factor V were present in the Ocplex factor concentrate. Addition of 1 $\mu$L stock solution of purified human factor V 5mg/mL was done, final concentration thus about 5 $\mu$g/mL.. INR=1.9. (Expected value about 2.0) If 120 $\mu$L of Ocplex was added to 1 mL 25 mmol/L HEPES-buffer pH 7.4 (without calcium followed by 1+3 mixture of calcium-containing (8.8 mmol/L) low ionic strength HEPES-buffer followed by 1 $\mu$L purified human factor V INR was 1.4. For stability at room temperature of different mixtures see table 12 below.

**Table 12 - Stability data**

| Ocplex addition | FV addition | Day=0 | 1 | 2 | 6 | 7 | 12 |
|---|---|---|---|---|---|---|---|
| 40 µL | no | 7.3 | 5.8 | 6.5 | >8 | nd | error |
| 120 µL | no | 2.6 | 2.3 | 2.2 | 2.1 | nd | 2.5 |
| 40 µL | 1 µL | 1.9 | 1.8 | 1.9 | 2.0 | 2.1 | nd |
| 120 µL | 1 µL | 1.4 | 1.2 | 1.2 | 1.3 | 1.3 | nd |

Obviously purified coagulation factors II, VII and X (Ocplex) in calcium-containing HEPES-buffer could be used as a ready to use liquid control material for point-of-care instruments for prothrombin time.

*Exampe 11 - Prolonged stability test with plasma made deficient in contact activating factors utilizing titanium dioxide*

All variants in this experiment included deficient plasma produced as described in for titanium dioxide in section "Production of FXII-deficient plasma using absorption to different negatively charged materials and comparison of stability of deficient plasma obtained by such absorption" then diluted 1+3 l low ionic strength HEPES-buffer with calcium to get a final Ca-concentration of about 5.6 mmol/L. Three variants were tested, without any further addition of 10 mmol/L sodium azide or with Berinert® (CSL Behring Ltd, it is plasma derived human C-1-estarase inhibitor, it is known to inhibit FXIIA, here used in order to inactivate any eventually remaining FXII/FXIIa).with a final concentration 5 of U/mL Storage was at room temperature. The results are seen in Table 13 and Table 14.

**Table 13 - Stability data**

| Variant | Day 0 | 1 | 2 | 3 | 6 | 8 | 10 | 36 | 50 |
|---|---|---|---|---|---|---|---|---|---|
| Plain | 1.8 | 1.8 | 1.8 | 1.9 | 2.0 | 2.0 | 2.1 | 2.2 | 2.6 |
| Azide | 1.9 | 1.9 | 1.9 | 1.9 | 2.0 | 2.0 | 2.1 | 2.2 | 2.3 |
| Berinert® | 1.8 | 1.8 | 1.9 | 1.9 | 2.0 | 2.0 | 2.1 | 2.3 | 2.7 |

In conclusion Berinert® did not improve stability, but sodium azide did. Stability at room temperature with an INR change less than 15% from day 0 is 36 days. Stability was not seen up to day 50.

**Table 14 - Variant with azide (not same batch as above) and a comparison of stability at room temperature vs +4C.**

| Temp | Day 0 | 1 | 2 | 5 | 8 | 18 | 28 | 35 | 44 | 58 | 86 |
|---|---|---|---|---|---|---|---|---|---|---|---|
| RT | 1.7 | 1.8 | 1.8 | 1.8 | 1.9 | 2.0 | 2.1 | 2.1 | 2.1 | 2.3 | 2.4 |
| +4C | - | 1.8 | - | - | - | - | 2.1 | 2.1 | 2.1 | nd | 2.0 |

INR change less than 15% until day 44 at +4C. More experiments needed to check for stability at +4C beyond 86 days. Deficient plasma diluted 1+3 in low ionic strength HEPES-buffer with calcium as above was lyophilized after storage 86 days at room temperature, after reconstitution with water INR was 2.3, one day later stored at room temperature same value, i.e. INR= 2.3.

The concentration of factor XII in the plasma after absorption with titanium dioxide was measured using a one-stage clotting assay on ACL.

*Example 12 - Plasma from donor with hereditary FXII-deficiency. Titration of lowest possible final concentration of ionized calcium to get a stable INR on Coaguchek XS*

The blood was collected in 1/10 volume 0.13 mol/L citrate. The deficient plasma was diluted 1+3 in buffer a low ionic strength HEPES- buffer (25 mmol/L, pH 7.4) adding different concentrations of $CaCl_2$, the low ionic strength in order to be able to use the Coaguchek XS instrument with code chip and test strips with intended use for native human capillary blood (Impedance measurement is used in the instrument to check if sample is blood or plasma, if not as expected the instrument gives no result but an error message. We wanted to use the same procedure as for whole blood, hence the use of this buffer.) The expected INR-value is 1.9.

To 1425 µL low ionic strength HEPES- buffer (25 mmol/L, pH 7.4) 75 µL 0.5 mol/L $CaCl_2$ was added, to 1450

μL 50 μL 0.5 mol/L CaCl2 to get same final volume, see table 15 below. Ionized calcium was measured in this mixture, the final concentration will be 75% thereof.

**Table 15 - Titration to elucidate the lowest possible concentration of ionised calcium compatible with obtaining a measureable INR on Couaguchek XS**

| Volume CaCl$_2$ | INR | Measured Ionized Ca$^{2+}$ |
| --- | --- | --- |
| 100 | 1.9 | Nd |
| 75 | 1.6 | Nd |
| 50 | 1.3 | Nd |
| 25 | 1.3 | 0.99 |
| 10 | >8 | Nd |

Even at 0.99 mmol/L ionized calcium an INR-value was obtained, although lower than expected. Thus lower calcium concentrations in the dilution buffer may be used, albeit the INR-value will deviate from the value expected from the dilution of plasma.

New stability test with a new donor, see table 16 below for results. The deficient plasma was diluted 1+3 in buffer a low ionic strength HEPES- buffer (25 mmol/L, pH 7.4) containing 8.8 mmol/L CaCl2 with 10 mmol/L sodium azide.

**Table 16 - Stability test in low ionic strength HEPES**

| Plasma from deficient donor | INR at time zero | INR day 1 | INR day 2 |
| --- | --- | --- | --- |
| FXII | 1.9 | 1.8 | 2.0 |

*Example 13 - Commercial, citrated FXII-deficient plasma from Precision Biologic*

The plasma, a commercially available plasma from a FXII-deficient patient, measured as sample with no additives on Coaguchek XS gave the result - Error (as expected, almost no free calcium)

Measured in 1+3 mixture with HEPES-buffer as above, see table 17 below for stability test.

**Table 17 - INR-values of FXII-deficient plasma, commercial and citrated with no additives upon use**

| | Day 0 | Day 1 | Day 2 | Day 5 |
| --- | --- | --- | --- | --- |
| INR | 2.1 | 2.5* | 2.6* | 2.9* |

* clot visible in the mixture.

Results gave a visible clot already at day 1 disqualifies the material.

*Example 14 - Addition of a FXIIa inhibitor to normal human plasma*

To citrated plasma (13 mmol/L citrate) corn trypsin inhibitor (CTI) from Hematologic Technologies Inc (Essex Junction, Vermont, US) was added to a final concentration of 50 μg/mL. Then the plasma was mixed 1+3 with the low ionic strength HEPES-buffer with calcium. INR-value on Coaguchek XS day = 0 was 1.9 (as expected) and on day=2 INR was 2.4. The inhibitor works but the stability was thus not sufficient, probably the CTI concentration must be higher.

*Example 15 - Absorption of contact activating factors using different negatively charged materials and metal oxides.*

Pooled platelet-free plasma from three donors was incubated for 10 minutes in different oxide powders. The oxide powder was mixed with plasma at a ratio of 1 mL powder/ 1 mL plasma. All oxides and kaolin were purchased from Sigma-Aldrich Sweden AB (Stockholm, Sweden). The test tubes containing the oxide/plasma mix was thereafter centrifuged at 2000 g for 4 minutes and the plasma supernatant transferred into new test tubes. The plasma was mixed with 25 mmol/L HEPES-buffer, pH 7.4 with 33 mmol/L calcium and stored at room temperature. 15 microliter from each test tube was measured in the Coaguchek XS Plus, the same day and in the following three days. Results are seen in Table 18.

**Table 18 - Test of different negatively charged material for absorption**

| Material | Day 0 | Day 1 | Day 2 | Day 3 | Day 6 |
|---|---|---|---|---|---|
| Silicon dioxide (silica) | 6.2 | 3.0 | 3.4 | 3.6 | ‡ |
| Chromium (III) oxide | † | † | Nd | nd | nd |
| Zirconium (IV) oxide | † | ‡ | ‡ | ‡ | nd |
| Manganese (IV) oxide | † | † | Nd | nd | nd |
| Titanium dioxide | 2.6 | 1.7 | 1.8 | 1.8 | 1.9 |
| Kaolin | † | † | Nd | † | nd |
| Iron (III) oxide | † | † | Nd | nd | nd |
| Aluminum oxide | 3.6 | 1.9 | 1.9 | 2.0 | 2.2 |
| † Error code 7, ‡ INR> 8 | | | | | |

In conclusion the only metal oxide besides titanium dioxide showing some promising results was aluminum oxide.

REFERENCES

1. Proctor RR and Rapaport SI. The partial thromboplastin time with kaolin. A simple screening test for first stage plasma clotting factor deficiencies. Am J Clin Pathol 1961; 36: 212-9.

2. Hattersley PG. Activated coagulation time of whole blood. JAMA 1966; 196: 436-40.

3. Machin D and Devine P. The effect of temperature and aprotinin during cardiopulmonary bypass on three different methods of activated clotting time measurement. J Extra Corpor Technol 2005; 37: 265-71.

4. Bosch YP, Ganushchak YM, and de Jong DS. Comparison of ACT point-of-care measurements: repeatability and agreement. Perfusion 2006; 21: 27-31.

5. Quick A. The prothrombin time in hemophilia and in obstructive jaundice. J Biol Chem 1935; 109: 73-4.

6. Quick A, Stanley-Brown M, and Bancroft F. A study of the coagulation defect in hemophilia and jaundice. Am J Med Sci 1935; 190: 501-11.

7. Owren PA. Thrombotest. A new method for controlling anticoagulant therapy. Lancet 1959; 2: 754-8.

8. Owren PA and Aas K. The control of dicumarol therapy and the quantitative determination of prothrombin and proconvertin. Scand J Clin Lab Invest 1951; 3: 201-8.

9. Mattsson C, Menschiek-Lundin A, Wåhlander K, and Lindahl TL. Effect of melagatran on prothrombin time assays depends on the sensitivity of the thromboplastin and the final dilution of the plasma sample. Thromb Haemost 2001; 86: 611-5.

10. Tripodi A, Chantarangkul V, Clerici M, Negri B, Galli M, and Mannucci PM. Laboratory control of oral anticoagulant treatment by the INR system in patients with the antiphospholipid syndrome and lupus anticoagulant. Results of a collaborative study involving nine commercial thromboplastins. Br J Haematol 2001; 115: 672-8.

11. Poller L and Hirsh JE. Oral Anticoagulants. ed 1. 1996, London: Arnold Publishers.

12. WHO, Expert Committee on Biological Standardisation 33th Report., in WHO Technical Report Series. 1983. p. 81-105.

13. Lindahl TL, Egberg N, Hillarp A, Odegaard OR, Edlund B, Svensson J, Sandset PM, and Rånby M. INR calibration of Owren-type prothrombin time based on the relationship between PT% and INR utilizing normal plasma samples. Thromb Haemost 2004; 91: 1223-31.

14. Hillarp A, Egberg N, Nordin G, Stigendal L, Fagerberg I, and Lindahl TL. Local INR calibration of the Owren type prothrombin assay greatly improves the intra- and interlaboratory variation. A three-year follow-up from the Swedish national external quality assessment scheme. Thromb Haemost 2004; 91: 300-7.

15. Kynde K, Henriksen G, Lindahl T, Örnemark U, Nordin G, Nilsson E, Torsteinsdóttir I, Pakkanen A, Oikkanen M, Stavelin A, and Steensland H. Measurement of P-Coagulation, Tissue Factor-induced; Relative Time Expressed as International Normalized Ratio (INR) - A Nordic Comparison. EQAnord Report 26 August 2005.

**Claims**

1. A stable solution comprising isolated factor II, VII and X and isolated plasma, wherein said factors are isolated in said isolated plasma and wherein said isolated plasma is depleted of coagulation factor XII, wherein the solution comprises ≥0.5 mmol/L and ≤ 20 mmol/L calcium and wherein said solution is stable, i.e. INR change of less than 15 %, for >4h at 2-8 °C and/or at RT.

2. The stable solution according to claim 1, wherein the depletion of coagulation factor XII is 95-100%.

3. The stable solution according to any of claims 1-2, with the proviso that said solution does not comprise any calcium chelating agent.

4. The stabile solution according to any of claims 1-3, wherein the plasma is depleted of platelets.

5. The stable solution according to any of claims 1-4, further comprising fibrinogen.

6. The stable solution according to any of claims 1-5, further comprising isolated and washed erythrocytes.

7. The stable solution according to any of claims 1-6, wherein the isolated plasma is mammalian plasma, human plasma or avian plasma.

8. The stable solution according to any of claims 1-7, wherein further contact activation factors are depleted, which factors are selected from the group consisting of prekallekrein, HMW kininogen, Factor V, Factor XI.

9. Use of the stable solution according to any of claims 1-8 as control material for coagulation analysis in assays/tests selected from a group consisting of prothrombin time (PT), mixing test, coagulation factor assays, thromboelastography euglobulin lysis time (ELT) or activated partial thromboplastin time (aPTT) with the proviso that no additional calcium is added to the solution upon use.

10. A method of producing a stable solution, the method comprising

    a) depleting isolated plasma of factor XII
    b) adjusting calcium levels to ≥ 0.5 mmol/L and ≤ 20 mmol/L.

11. The method according to claim 10, wherein the depletion of factor XII is done by using a metal oxide, such as titanium dioxide.

12. The method according to any of claims 10-11, further comprising depletion of platelets in the plasma.

13. The method according to any of claims 10-11, further comprising adding of washed eryth rocytes.

14. The method according to any of claims 10-13, wherein the plasma is human plasma or avian plasma.

15. A method of producing a stable solution, the method comprising the steps of

    a) adding isolated human factor II, VII, and X to isolated plasma depleted of coagulation factor XII, and
    b) adjusting levels of free calcium to ≥ 0.5 mmol/L and ≤ 20 mmol/L

16. The method according to claim 15, wherein the isolated plasma is mammalian non-human plasma or avian plasma.

17. The method according to any of claims 15-16, further comprising depleting the mammalian non-human plasma of at least one contact-activating factor.

18. A stable solution according to any of claims 1-8, obtainable by the methods according to any of claims 10-17.

19. A kit comprising the stable solution according to any of claims 1-8.

20. The kit according to claim 19, wherein the stable solution is provided in solution or in lyophilized form.

**Patentansprüche**

1. Stabile Lösung, welche die isolierten Faktoren II, VII und X sowie isoliertes Plasma umfasst, wobei die Faktoren in dem isolierten Plasma isoliert vorliegen und wobei das isolierte Plasma bezüglich des Gerinnungsfaktors XII abgereichert ist, wobei die Lösung ≥ 0,5 mmol/L und ≤ 20 mmol/L an Calcium umfasst und wobei die Lösung stabil ist, d.h. bei 2 bis 8 °C und/oder bei RT eine INR-Veränderung zeigt, die > 4 Std. lang weniger als 15 % beträgt.

2. Stabile Lösung nach Anspruch 1, wobei die Abreicherung bezüglich des Gerinnungsfaktors XII 95 bis 100 % beträgt.

3. Stabile Lösung nach einem beliebigen der Ansprüche 1 bis 2, mit der Maßgabe, dass die Lösung keinerlei Mittel umfasst, das mit Calcium eine Chelatverbindung bildet.

4. Stabile Lösung nach einem beliebigen der Ansprüche 1 bis 3, wobei das Plasma bezüglich der Blutplättchen abgereichert ist.

5. Stabile Lösung nach einem beliebigen der Ansprüche 1 bis 4, welche weiterhin Fibrinogen umfasst.

6. Stabile Lösung nach einem beliebigen der Ansprüche 1 bis 5, welche weiterhin isolierte und gewaschene Erythrozyten umfasst.

7. Stabile Lösung nach einem beliebigen der Ansprüche 1 bis 6, wobei es sich bei dem isolierten Plasma um Plasma von Säugetieren, Plasma des Menschen oder Plasma von Vögeln handelt.

8. Stabile Lösung nach einem beliebigen der Ansprüche 1 bis 7, welche bezüglich weiterer Kontakt-Aktivierungsfaktoren abgereichert ist, wobei diese Faktoren aus der Gruppe ausgewählt sind, die aus Präkallekrein, HMW-Kininogen, dem Faktor V, dem Faktor XI besteht.

9. Verwendung der stabilen Lösung nach einem beliebigen der Ansprüche 1 bis 8, als Kontrollmaterial für die Gerinnungsanalyse in Versuchsreihen/Prüfungen, die aus einer Gruppe ausgewählt sind, welche aus der Thromboplastinzeit (TPZ), dem Mischtest, den Gerinnungsfaktor-Versuchsreihen, der thromboelastographisch bestimmten Euglobulin-Lysezeit (ELT) oder der aktivierten partiellen Thromboplastinzeit (aPPT) besteht, mit der Maßgabe, dass der Lösung bei ihrer Verwendung kein zusätzliches Calcium beigefügt wird.

10. Verfahren zur Herstellung einer stabilen Lösung, wobei das Verfahren Folgendes umfasst

    a) Abreichern des isolierten Plasmas bezüglich des Faktors XII
    b) Einstellen der Calciumspiegel auf ≥ 0,5 mmol/L und ≤ 20 mmol/L.

11. Verfahren nach Anspruch 10, wobei die Abreicherung bezüglich des Faktors XII unter Verwendung eines Metalloxids, wie etwa Titandioxid, erfolgt.

12. Verfahren nach einem beliebigen der Ansprüche 10 bis 11, welches weiterhin eine Abreicherung bezüglich der Blutplättchen in dem Plasma umfasst.

13. Verfahren nach einem beliebigen der Ansprüche 10 bis 11, welches weiterhin den Zusatz gewaschener Erythrozyten umfasst.

14. Verfahren nach einem beliebigen der Ansprüche 10 bis 13, wobei es sich bei dem Plasma um Plasma des Menschen oder Plasma von Vögeln handelt.

15. Verfahren zur Herstellung einer stabilen Lösung, wobei das Verfahren die folgenden Schritte umfasst

    a) Zusetzen der isolierten Faktoren II, VII und X des Menschen zu dem Plasma, welches bezüglich des Gerinnungsfaktors XII abgereichert ist, und
    b) Einstellen der Spiegel an freiem Calcium auf ≥ 0,5 mmol/L und ≤ 20 mmol/L.

16. Verfahren nach Anspruch 15, wobei es sich bei dem isolierten Plasma um Plasma von Säugetieren mit Ausnahme des Menschen oder um Plasma von Vögeln handelt.

**17.** Verfahren nach einem beliebigen der Ansprüche 15 bis 16, welches weiterhin die Abreicherung des Plasmas, das von Säugetieren mit Ausnahme des Menschen stammt, bezüglich mindestens eines kontakt-aktivierenden Faktors umfasst.

**18.** Stabile Lösung nach einem beliebigen der Ansprüche 1 bis 8, welche mittels der Verfahren nach einem beliebigen der Ansprüche 10 bis 17 erhalten werden kann.

**19.** Kit, welches die stabile Lösung nach einem beliebigen der Ansprüche 1 bis 8 umfasst.

**20.** Kit nach Anspruch 19, wobei die stabile Lösung in Form einer Lösung oder gefriergetrocknet bereitgestellt wird.

**Revendications**

**1.** Solution stable comprenant les facteurs isolés II, VII et X et le plasma isolé, dans laquelle lesdits facteurs sont isolés dans ledit plasma isolé et dans laquelle ledit plasma isolé est appauvri en facteur de coagulation XII, laquelle solution comprend $\geq$ 0,5 mmol/l et $\leq$ 20 mmol/l de calcium et ladite solution est stable, c'est-à-dire présente une variation du rapport international normalisé (RIN) inférieure à 15 %, pendant > 4 h entre 2 °C et 8 °C et/ou à température ambiante (RT).

**2.** Solution stable selon la revendication 1, dans laquelle l'appauvrissement du facteur de coagulation XII est compris entre 95 % et 100 %.

**3.** Solution stable selon l'une quelconque des revendications 1 et 2, à condition que ladite solution ne comprenne aucun chélateur du calcium.

**4.** Solution stable selon l'une quelconque des revendications 1 à 3, dans laquelle le plasma est appauvri en plaquettes.

**5.** Solution stable selon l'une quelconque des revendications 1 à 4, comprenant en outre un fibrinogène.

**6.** Solution stable selon l'une quelconque des revendications 1 à 5, comprenant en outre des érythrocytes isolés et lavés.

**7.** Solution stable selon l'une quelconque des revendications 1 à 6, dans laquelle le plasma isolé est le plasma mammifère, le plasma humain ou le plasma aviaire.

**8.** Solution stable selon l'une quelconque des revendications 1 à 7, dans laquelle les facteurs Fitzgerald sont appauvris, lesquels facteurs sont choisis dans le groupe constitué par la prékallikréine, le kininogène de haut poids moléculaire, le facteur V et le facteur XI.

**9.** Utilisation de la solution stable selon l'une quelconque des revendications 1 à 8 en tant que matériau de contrôle pour l'analyse de la coagulation dans les dosages/tests choisis dans un groupe constitué par le temps de prothrombine (PT), le test de mélange, les dosages du facteur de coagulation, le temps de lyse des euglobulines par thromboélastographie (ELT) ou le temps de thromboplastine partielle activée (aPTT) à condition qu'aucun autre calcium ne soit ajouté à la solution au moment de son utilisation.

**10.** Procédé de production d'une solution stable, le procédé comprenant les étapes consistant à :

a) appauvrir le plasma isolé du facteur XII,
b) ajuster les taux de calcium à $\geq$ 0,5 mmol/l et $\leq$ 20 mmol/l.

**11.** Procédé selon la revendication 10, dans lequel l'appauvrissement du facteur XII est effectué en utilisant un oxyde métallique tel que le dioxyde de titane.

**12.** Procédé selon l'une quelconque des revendications 10 et 11, comprenant en outre l'appauvrissement des plaquettes dans le plasma.

**13.** Procédé selon l'une quelconque des revendications 10 et 11, comprenant en outre l'ajout d'érythrocytes lavés.

**14.** Procédé selon l'une quelconque des revendications 10 à 13, dans lequel le plasma est le plasma humain ou le plasma aviaire.

**15.** Procédé de production d'une solution stable, le procédé comprenant les étapes consistant à :

a) ajouter les facteurs humains isolés II, VII et X au plasma isolé appauvri en facteur de coagulation XII, et
b) ajuster les taux de calcium libre à $\geq 0,5$ mmol/l et $\leq 20$ mmol/l.

**16.** Procédé selon la revendication 15, dans lequel le plasma isolé est le plasma non humain mammifère ou le plasma aviaire.

**17.** Procédé selon l'une quelconque des revendications 15 et 16, comprenant en outre l'appauvrissement du plasma non humain mammifère en au moins un facteur Fitzgerald.

**18.** Solution stable selon l'une quelconque des revendications 1 à 8, pouvant être obtenue par les procédés selon l'une quelconque des revendications 10 à 17.

**19.** Kit comprenant la solution stable selon l'une quelconque des revendications 1 à 8.

**20.** Kit selon la revendication 19, dans lequel la solution stable est fournie sous forme de solution ou sous forme lyophilisée.

Fig. 1

Fig. 2

Fig. 3

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 8911865 A
- WO 2008081025 A
- WO 9501571 A
- WO 2004047859 A

### Non-patent literature cited in the description

- **RAMSTRÖM, S.** *Thesis,* 2001, ISBN 91-7373-535-3
- **RAMSTRÖM, S.** Linköping University Medical Dissertation no. 776. *Thesis,* 2001, ISBN 91-7373-535-3
- **BRAAT et al.** *Eur. J. Biochemistry,* 1999, vol. 263, 904-911
- Current Protocols in Immunology. John Wiley and Sons, Inc, 2007
- **JONES.** *Blood Coagul Fibrinolysis,* 1998, vol. 9 (2), 183-7
- **STÜRZEBECHER, J. et al.** *Thromb Res.,* 1989, vol. 55 (6), 709-15
- **TANKERSLAY et al.** *Blood,* 1983, vol. 62, 488-456
- **HARLOW, E ; LANE, D.** Antibodies: A laboratory Manual. Cold Spring Harbor Laboratory Press, 1988
- **HARLOW ; LANE.** Antibodies: A Laboratory. Cold Spring Habor Laboratory Press
- **BETTER et al.** *Science,* 1988, vol. 240, 1041
- **SKERRA et al.** *Science,* 1988, vol. 240, 1038
- **BIRD et al.** *Science,* 1988, vol. 242, 423
- **HUSTON et al.** *Proc. Natl. Acad. Sd. USA,* 1988, vol. 85, 5879
- **WARD et al.** *Nature,* 1989, vol. 341, 544
- **WINTER ; MILSTEIN.** *Nature,* 1991, vol. 349, 293-299
- **ORLANDI et al.** *Proc. Natl. Acad. Sci. U.S.A.,* 1989, vol. 86, 3833-3837
- **WINTER et al.** *Nature,* 1991, vol. 349, 293-299
- **KOHLER et al.** *Nature,* 1975, vol. 256, 4950497
- **KOZBOR et al.** *J Immunol. Methods,* 1985, vol. 81, 31-42
- **COTE et al.** *Proc. Natl. Acad. Sci., USA,* 1983, vol. 80, 2026-2030
- **COLE et al.** *Mol Cell. Biol.,* 1984, vol. 62, 109-1 20
- **KOHLER ; MILSTEIN.** *Nature,* 1975, vol. 256, 495
- **ENGVALL.** *Enzymol.,* 1980, vol. 70, 419
- **H ZOLA.** Monoclonal Antibodies: A manual of techniques. CRC Press, 1988
- **J G R HURRELL.** Monoclonal Hybridoma Antibodies: Techniques and Applications. CRC Press, 1982
- Antibodies: A Laboratory Manual. Cold Spring Harbor Laboratory
- **HARLOW ; LANE.** Antibodies: A Laboratory Manual. Cold Spring Harbor Laboratory, 1988
- *Enzymol.,* 1980, vol. 70, 41-9
- **ZHUO R ; VOGLER EA.** Practical application of a chromogenic FXIIa assay. *Biomaterials,* October 2006, vol. 27 (28), 4840-5
- **JONES DW ; GALLIMORE MJ ; WINTER M.** An automated chromogenic peptide substrate assay for coagulation factor XII. *Blood Coagul Fibrinolysis,* March 1998, vol. 9 (2), 183-7
- Klinisk Biokemi i Norden. Laboratory techniques in thrombosis - a manual. 2008
- ACAT Assay procedures. Kluwer Academic Publishers, 2000
- **RÅNBY et al.** *Clinical Chemistry,* vol. 45 (8), 1176-1180
- Extracellular calcium sensing and extracellular calcium signalling. **LAURELLS KLINISK KEMI.** Physiol Rev. 2001, vol. 81, 239-97
- **LARSSON L ; MAGNUSSON P.** Ionized calcium or corrected total calcium?. *J Bone Miner Res,* 2003, vol. 18, 1554-5
- Extracellular calcium sensing and extracellular calcium signalling. **LAURELLS KLINISK KEMI.** Physiol Rev. 2001, vol. 81, 239-97
- **LINDAHL TL et al.** INR calibration of Owren-type prothrombin time based on the relationship between PT% and INR utilizing normal plasma samples. *Thromb Haemost,* 2004, vol. 91, 1224-31
- **PROCTOR RR ; RAPAPORT SI.** The partial thromboplastin time with kaolin. A simple screening test for first stage plasma clotting factor deficiencies. *Am J Clin Pathol,* 1961, vol. 36, 212-9
- **HATTERSLEY PG.** Activated coagulation time of whole blood. *JAMA,* 1966, vol. 196, 436-40

- **MACHIN D ; DEVINE P.** The effect of temperature and aprotinin during cardiopulmonary bypass on three different methods of activated clotting time measurement. *J Extra Corpor Technol,* 2005, vol. 37, 265-71
- **BOSCH YP ; GANUSHCHAK YM ; DE JONG DS.** Comparison of ACT point-of-care measurements: repeatability and agreement. *Perfusion,* 2006, vol. 21, 27-31
- **QUICK A.** The prothrombin time in hemophilia and in obstructive jaundice. *J Biol Chem,* 1935, vol. 109, 73-4
- **QUICK A ; STANLEY-BROWN M ; BANCROFT F.** A study of the coagulation defect in hemophilia and jaundice. *Am J Med Sci,* 1935, vol. 190, 501-11
- **OWREN PA. THROMBOTEST.** A new method for controlling anticoagulant therapy. *Lancet,* 1952, vol. 2, 754-8
- **OWREN PA ; AAS K.** The control of dicumarol therapy and the quantitative determination of prothrombin and proconvertin. *Scand J Clin Lab Invest,* 1951, vol. 3, 201-8
- **MATTSSON C ; MENSCHIEK-LUNDIN A ; WÅH-LANDER K ; LINDAHL TL.** Effect of melagatran on prothrombin time assays depends on the sensitivity of the thromboplastin and the final dilution of the plasma sample. *Thromb Haemost,* 2001, vol. 86, 611-5
- **TRIPODI A ; CHANTARANGKUL V ; CLERICI M ; NEGRI B ; GALLI M ; MANNUCCI PM.** Laboratory control of oral anticoagulant treatment by the INR system in patients with the antiphospholipid syndrome and lupus anticoagulant. Results of a collaborative study involving nine commercial thromboplastins. *Br J Haematol,* 2001, vol. 115, 672-8
- **POLLER L ; HIRSH JE.** Oral Anticoagulants. Arnold Publishers, 1966
- WHO, Expert Committee on Biological Standardisation 33th Report. *WHO Technical Report Series,* 1983, 81-105
- **LINDAHL TL ; EGBERG N ; HILLARP A ; ODE-GAARD OR ; EDLUND B ; SVENSSON J ; SAND-SET PM ; RÅNBY M.** INR calibration of Owren-type prothrombin time based on the relationship between PT% and INR utilizing normal plasma samples. *Thromb Haemost,* 2004, vol. 91, 1223-31
- **HILLARP A ; EGBERG N ; NORDIN G ; STIGEN-DAL L ; FAGERBERG I ; LINDAHL TL.** Local INR calibration of the Owren type prothrombin assay greatly improves the intra- and interlaboratory variation. A three-year follow-up from the Swedish national external quality assessment scheme. *Thromb Haemost,* 2004, vol. 91, 300-7
- **KYNDE K ; HENRIKSEN G ; LINDAHL T ; ÖRNE-MARK U ; NORDIN G ; NILSSON E ; TORSTEINSDÓTTIR I ; PAKKANEN A ; OIKKANEN M ; STAVELIN A.** Measurement of P-Coagulation, Tissue Factor-induced; Relative Time Expressed as International Normalized Ratio (INR) - A Nordic Comparison. *EQAnord Report,* 26 August 2005